# EUROPEAN PATENT SPECIFICATION

(11) **EP 2 020 203 B1**
(45) Date of publication and mention of the grant of the patent: **27.08.2014**
(21) Application number: 08013678.1
(22) Date of filing: 30.07.2008
(51) Int. Cl.: A61B 1/04, A61B 5/06, A61B 8/12, G06F 19/00

(54) **Medical guiding system**
Medizinisches Führungssystem
Système de guidage médical

(30) Priority: 31.07.2007 JP 2007199744
(43) Date of publication of application: 04.02.2009
(73) Proprietor: Olympus Medical Systems Corp., Tokyo 151-0072 (JP)
(72) Inventor: Ikuma, Soichi, Tokyo 151-0072 (JP); Kawashima, Tomonao, Tokyo 151-0072 (JP); Komuro, Masahiko, Tokyo 151-0072 (JP)
(74) Representative: von Hellfeld, Axel

(56) References cited:
- EP-A1- 1 504 721
- WO-A1-2006/057296

## Description

### BACKGROUND OF THE INVENTION

### 1. Field of the Invention

The present invention relates to a medical guiding system for creating a guide image to indicate a position of an ultrasound tomographic image with respect to an organ.

### 2. Description of Related Art

Conventionally, in ultrasound diagnostic apparatuses, techniques have been proposed to enable an operator or the like to recognize which region of the subject was scanned to acquire the current ultrasound tomographic image and the recorded ultrasound tomographic image which were generated using the ultrasound transducer.

For example, Japanese Patent Application Laid-Open Publication No. 2004-121488 discloses a technique to arrange and display on a three-dimensional coordinates ultrasound tomographic images acquired using an ultrasound probe equipped with a position sensor during inspection. Furthermore, Japanese Patent Application Laid-Open Publication No. 2006-149481 discloses a technique in which an ultrasound probe equipped with a position sensor is used and a position of ultrasound tomographic image detected by the position sensor is displayed as an ultrasound tomographic image marker on a three-dimensional image created from a pattern diagram data of a human body in a synthesized manner.

In the technique to arrange and display the ultrasound tomographic images on the three-dimensional coordinates, which is described in the Japanese Patent Application Laid-Open Publication No. 2004-121488, an operator cannot know the positional relationship between the ultrasound tomographic images and an organ until he or she interprets the ultrasound tomographic images using the positional relationship among the series of ultrasound tomographic images arranged on the three-dimensional coordinates as an aid. Therefore, in order to confirm whether or not inspection has been successfully performed on an entirety of a region to be inspected without making an oversight, an operator is required to create volume data from acquired ultrasound tomographic images to interpret the images. As a result, there has been a problem of longer inspection time.

In addition, in the method for displaying the ultrasound tomographic image marker on the three-dimensional image in a synthesized manner, which is described in Japanese Patent Application Laid-Open Publication No. 2006-149481, only the position of one displayed ultrasound tomographic image is displayed, so that an operator cannot confirm the range which has been inspected from the start of the ultrasound inspection until now. Therefore, the operator cannot determine the range on which the ultrasound inspection has been already performed during the inspection, so that it has been difficult to inspect the entirety of the region to be inspected without oversight in a short time.

The present invention has been achieved in view of the above circumstances, and an object of the present invention is to provide a medical guiding system for allowing an operator to easily confirm, during inspection or after inspection, a range on which ultrasound scan has been performed.

WO 2006/057296 A1 discloses an ultrasonic diagnostic apparatus comprising a receiving coil serving as a body cavity sample point position detection means. A position vector of the receiving coil is considered as the position vector at the rotating center of an ultrasonic transducer. An ultrasonic observation unit drives the ultrasonic transducer and creates a single digitized ultrasonic tomographic image data. The diagnostic apparatus further comprises reference image data storage means that stores reference image data such as anatomical image information, a 3D guide image forming circuit serving as 3D guide image forming means and a display unit for displaying the ultrasonic tomographic image data. The 3D guide image includes the reference image data and the ultrasonic tomographic image marker.

EP 1 504 721 A1 discloses an ultrasonic diagnostic apparatus comprising a position detecting portion for detecting the position of a send coil, an image constructing circuit and a display circuit for performing digital-analog converting processing on image data having undergone different kinds of images processing in the image processing circuit to convert analog video signals and causing a monitor to display an image. The displayed image includes a magnetic sensor unit marker, plural ultrasonic image markers displayed with respect to the magnetic sensor unit marker, a direction marker displayed on the ultrasonic image marker and a locus marker.

### SUMMARY OF THE INVENTION

A medical guiding system according to claim 1 is provided.

The above and other objects, features and advantages of the invention will become more clearly understood from the following description referring to the accompanying drawings.

### BRIEF DESCRIPTION OF THE DRAWINGS

FIG. 1 is a view showing a configuration of a medical guiding system.
FIG. 2 is a view showing four body surface detection coils mounted on sample points of a subject.
FIG. 3 is a view conceptually showing reference image data stored in a reference image storage portion.
FIG. 4 is a view showing a configuration of a voxel space.
FIG. 5 is a flowchart of a main routine showing an entire action of the medical guiding system.
FIG. 6 is a flowchart showing a display processing of an ultrasound tomographic image and a three-dimensional guide image.
FIG. 7 is a table showing one example of image scan region data.
FIG. 8 is a view describing the three-dimensional guide image.
FIG. 9 is a view describing mixed data displayed on a display apparatus.
FIG. 10 is a table showing one example of files saved in a tomographic-image-for-saving storage portion.
FIG. 11 is a flowchart showing a creation processing of three-dimensional guide image data.
FIG. 12 is one example of a three-dimensional model image of an organ of interest.
FIG. 13 is a view describing the concept of coordinate transformation.
FIG. 14 is a view describing an image scan history reproduced image.
FIG. 15 is a view describing an image scan history marker according to a second embodiment.
FIG. 16 is a view describing an image scan history marker according to a third embodiment.

### DETAILED DESCRIPTION OF PREFERRED EMBODIMENTS

Hereinafter, embodiments of the present invention will be described with reference to the drawings. In the embodiments described below, description will be made assuming that an ultrasound endoscope is used as a medical instrument, and a medical guiding system creates a guide image to support operation of the ultrasound endoscope. Note that the present invention is not limited to the shape, the size ratio, or arranging position of each of the components and the like shown in the drawings.

### (First Embodiment)

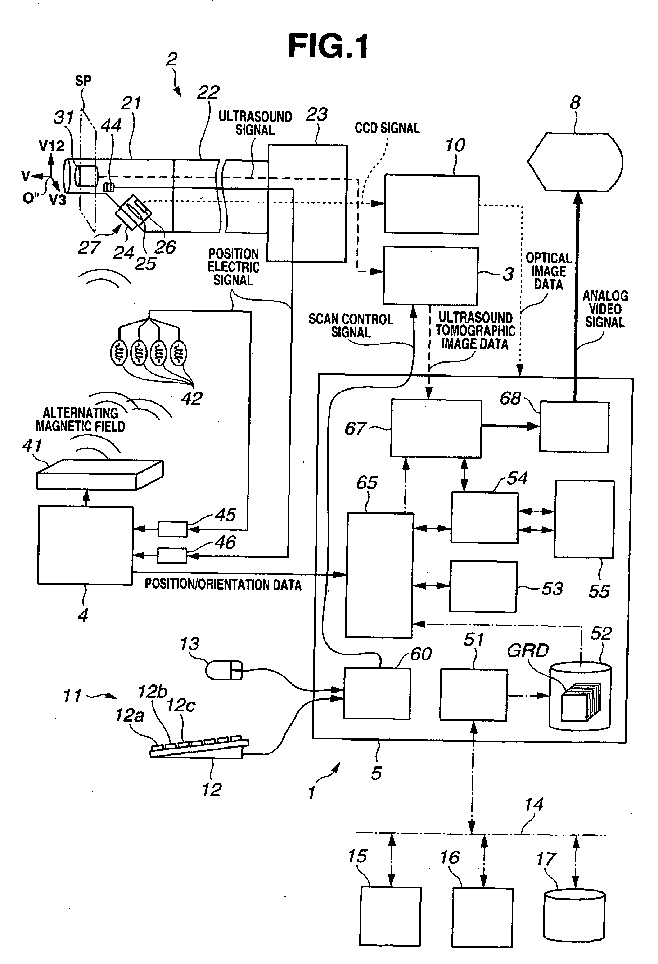

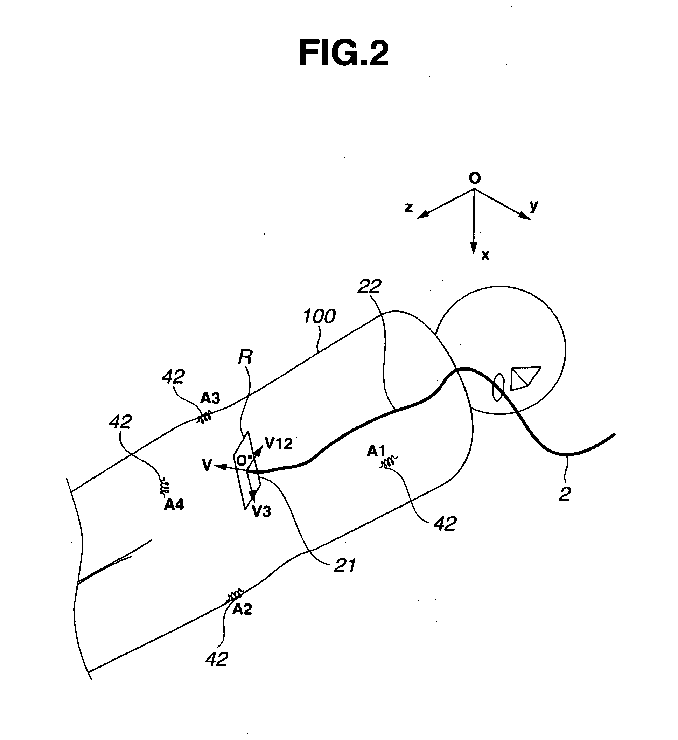

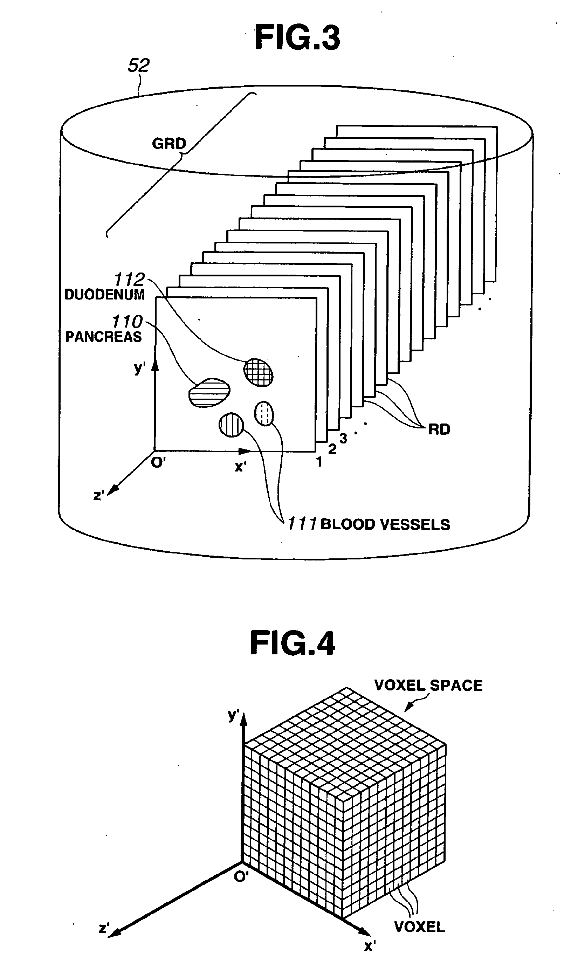

Hereinafter, a first embodiment of the present invention is described with reference to FIGS. 1 to 14. FIG. 1 is a block diagram showing a configuration of a medical guiding system. FIG 2 is a view showing four body surface detection coils mounted on sample points of a subject. FIG 3 is a view conceptually showing reference image data stored in a reference image storage portion. FIG. 4 is a view showing a configuration of a voxel space.

A medical guiding system 1 of the present embodiment is a system for performing a guide to support diagnosis operation by an ultrasound endoscope 2 as a medical instrument. The medical guiding system 1 of the present embodiment includes the ultrasound endoscope 2, an ultrasound observation apparatus 3, a position/orientation calculation apparatus 4, an image processing apparatus 5, a display apparatus 8, an optical observation apparatus 10, and an input apparatus 11. The apparatuses are connected with each other by wired or wireless communication means.

In addition, the medical guiding system 1 is connected to a network 14 provided outside of the medical guiding system 1, which uses an optical fiber, an electrical cable, or wireless communication and the like. The network 14 is connected with an X-ray three-dimensional helical CT apparatus (X-ray three-dimensional computed tomography system) 16, a three-dimensional MRI apparatus (three-dimensional magnetic resonance imaging system) 15, and an external storage apparatus 17. The medical guiding system 1 is capable of transmitting and receiving data to and from the three-dimensional MRI apparatus 15, the X-ray three-dimensional helical CT apparatus 16, and the external storage apparatus 17, via the network 14.

The ultrasound endoscope 2 as a medical instrument includes: a rigid portion 21 configured of a rigid material such as stainless steel and disposed at a distal end of an insertion portion to be inserted into a body inside such as an esophagus, a stomach, and a duodenum; a long flexible portion 22 configured of a flexible material and disposed on a proximal end side of the rigid portion 21; and an operation portion 23 configured of a rigid material and disposed on a proximal end side of the flexible portion 22. An operator grasps the operation portion 23 to operate the ultrasound endoscope 2. In addition, the ultrasound endoscope 2 is electrically connected to the ultrasound observation apparatus 3, the optical observation apparatus 10, and the position/orientation calculation apparatus 4 via a connector not shown.

The rigid portion 21 includes an image pickup apparatus 27, an ultrasound transducer array 31, and an image position/orientation detection coil 44. The image pickup apparatus 27 includes: a CCD (Charge Coupled Device) 26 as an image pickup device; a lens 25 for forming an image of a subject on a light-receiving surface of the CCD 26; and the like, and serves as an apparatus for optically picking up an image of inside of the body through an optical observation window 24 made of glass. The rigid portion 21 is provided with an illumination apparatus, not shown, for irradiating illumination light in a field of view direction of the image pickup apparatus 27. The image pickup apparatus 27 acquires an optical image of the body inside illuminated by the illumination apparatus to output the acquired image as a CCD signal to the optical observation apparatus 10.

The ultrasound transducer array 31 is a so-called electronic radial scan ultrasound transducer array and configured of a plurality of ultrasound transducers annularly disposed around an insertion axis along an insertion direction of the rigid portion 21 into the body. The ultrasound transducer array 31 transmits and receives an ultrasound beam while scanning in a radiation direction at a predetermined time interval on a plane orthogonal to the insertion axis to acquire an echo signal required for acquiring an ultrasound tomographic image, and outputs the acquired echo signal to the ultrasound observation apparatus 3.

The ultrasound transducer array 31 is electrically connected to the ultrasound observation apparatus 3. The ultrasound observation apparatus 3 controls intensity, angle, focus, and the like of the ultrasound beam transmitted by the ultrasound transducer array 31, and also generates ultrasound tomographic image data on a scanning surface based on the echo signal acquired by the ultrasound transducer array 31 and outputs the generated ultrasound tomographic image data.

Note that a coordinate system fixed in a real space is defined as orthogonal coordinate axes O-xyz below. In the present embodiment, as shown in FIG. 2 for example, the position of the orthogonal coordinate axes O-xyz is fixed with respect to an examination table on which a subject 100 lies at the time of ultrasound diagnosis.

In addition, a plane to be scanned by the ultrasound transducer array 31 is called as a radial scanning surface SP. Furthermore, orthonormal bases (unit vectors of the respective directions) V, V3, and V12 fixed to the rigid portion 21 are defined as shown in FIG. 1. That is, in a case where the base vector V is assumed to be parallel to an insertion axis direction of the rigid portion 21, in other words, a normal direction vector of the radial scanning surface SP, and a predetermined scan direction of the ultrasound transducer array 31 is assumed to be the twelve o'clock direction, the base vector to orient in the three o'clock direction is defined as V3, and the base vector to orient in the twelve o'clock direction is defined as V12. Note that, though vectors are normally described in bold italics, the vectors are denoted in normal alphanumeric characters in the present embodiment. In addition, a scanning range by the ultrasound transducer array 31 on the surface to be scanned by the ultrasound transducer array is referred to as an image scan range R of ultrasound tomographic image data.

The image position/orientation detection coil 44 is disposed in the rigid portion 21 so as to be located in the vicinity of the center of the circular ultrasound transducer array 31 with the positional relationship with respect to the ultrasound transducer array 31 fixed. The image position/orientation detection coil 44 includes integrally formed two coils respectively wound around the axes parallel to the base vectors V and V3. In addition, the center of the image position/orientation detection coil 44 is defined as a position O". Note that, in FIG. 1, the image position/orientation detection coil 44 is shown apart from the center of the ultrasound transducer array 31 for the convenience of description.

That is, the position O" of the image position/orientation detection coil 44 generally coincides with a scanning center position of the ultrasound transducer array 31, and indicates a center position of the image scan range R of ultrasound tomographic image data in the present embodiment. In addition, the base vectors V, V3, and V12 indicate an orientation of ultrasound tomographic image data.

The position/orientation calculation apparatus 4 as a detection portion is connected to the image position/orientation detection coil 44 disposed in the rigid portion 21 of the ultrasound endoscope 2 via an A/D conversion apparatus 46 and is also electrically connected to a transmission antenna 41. The position/orientation calculation apparatus 4 is connected to body surface detection coils 42 via an A/D conversion apparatus 45. Moreover, the position/orientation calculation apparatus 4 is electrically connected to an image processing apparatus 5 via a cable of the RS-232C standard, for example.

The transmission antenna 41 includes a plurality of magnetic field generation coils of different winding axes orientation, not shown, and generates an alternating magnetic field in response to an output from the position/orientation calculation apparatus 4.

The transmission antenna 41 is arranged with the position thereof being fixed in a real space. In the present embodiment, the transmission antenna 41 is arranged such that the position thereof is fixed with respect to the examination table on which the subject 100 lies at the time of ultrasound diagnosis. That is, in the present embodiment, the position of the transmission antenna 41 is fixed on the orthogonal coordinate axes O-xyz.

The A/D conversion apparatuses 45, 46 include an amplifier, not shown, for amplifying an inputted signal, and an analog-digital conversion circuit, not shown, for sampling the amplified signal to convert the signal into digital data.

The image position/orientation detection coil 44 disposed in the rigid portion 21 of the ultrasound endoscope 2 detects the alternating magnetic field generated by the transmission antenna 41 and converts the detected alternating magnetic field into an position electric signal to output the position electric signal to the A/D conversion apparatus 46.

In addition, the plurality of body surface detection coils 42 respectively include coils for detecting the alternating magnetic field generated by the transmission antenna 41 and convert the alternating magnetic field detected by the coils into a position electric signals to output the position electric signals to the A/D conversion apparatus 45.

The position/orientation calculation apparatus 4 calculates the respective positions and the orientations of the image position/orientation detection coil 44 and the plurality of body surface detection coils 42 with respect to the transmission antenna 41, based on the position electric signals respectively outputted from the image position/orientation detection coil 44 and the body surface detection coils 42 and converted into digital data by the A/D conversion apparatuses 45, 46.

Specifically, the position/orientation calculation apparatus 4 calculates the center position O" of the image position/orientation detection coil 44 and the base vectors V and V3 on the orthogonal coordinate axes O-xyz, based on the position electric signal outputted from the image position/orientation detection coil 44 composed of two coils respectively wound around axes parallel to the base vectors V and V3, to output the information on the center position and base vectors to the image processing apparatus 5.

Furthermore, the position/orientation calculation apparatus 4 calculates the positions and orientations of the body surface detection coils 42 on the orthogonal coordinate axes O-xyz, based on the position electric signals outputted from the body surface detection coils 42, to output the information on the positions and orientations to the image processing apparatus 5.

In the present embodiment, four body surface detection coils 42 are mounted and fixed in the vicinity of four characteristic points on the body surface of the subject 100 (hereinafter, characteristic points on the body surface of the subject 100 are simply referred as sample points) by a tape, a belt, a band, an adhesive, negative pressure absorption, or the like.

Note that four sample points A1 to A4 are defined as follows in the present embodiment. That is, the sample point A1 is defined as "xiphoid process", the sample point A2 as "left anterior superior iliac spine" on the left side of the pelvis, the sample point A3 as "right anterior superior iliac spine" on the right side of the pelvis, and the sample point A4 as "spinous process of vertebral body" in the middle of the left and right anterior superior iliac spines on the spine (see FIG. 2).

As the four sample points A1 to A4, points which are not located on the same plane are adopted. Therefore, as shown in FIG. 2, it is possible to calculate an oblique coordinate system with the xiphoid process (the sample point A1) set as the origin and with the three vectors directed to other sample points A2 to A4 as fundamental vectors, on the orthogonal coordinate axes O-xyz.

Note that the four sample points A1 to A4 are not limited to those shown in the present embodiment, and appropriately changed depending on a configuration of a medical instrument to be used or on a region with respect to which the medical instrument is used. In addition, it is preferable that the four sample points A1 to A4 are characteristic points on the skeleton of the subject 100 and the positions thereof can be specified by the operator's palpation.

The calculation of the position O" of the image position/orientation detection coil 44 and the base vectors V, V3 and the calculation of the positions and the orientations of the four body surface detection coils 42 are performed by the position/orientation calculation apparatus 4 synchronously with the timing of acquiring ultrasound tomographic image data by the ultrasound observation apparatus 3.

Hereinafter, among the data calculated by the position/orientation calculation apparatus 4 and outputted to the image processing apparatus 5, the x-, y- and z-coordinate values of the position O" of the image position/orientation detection coil 44, the x-, y- and z-coordinate components of the base vector V, the x-, y- and z-coordinate components of the base vector V3 on the orthogonal coordinate axes O-xyz are referred to as "image position/orientation detection coil position/orientation data".

In addition, among the data calculated by the position/orientation calculation apparatus 4 and outputted to the image processing apparatus 5, the x-, y- and z-coordinate values of each of the four body surface detection coils 42 on the orthogonal coordinate axes O-xyz are referred to as "body surface detection coil position data".

Furthermore, the data acquired by combining "the image position/orientation detection coil position/orientation data" and the "body surface detection coil position data" is only referred to as "position/orientation data".

Next, the configuration of the image processing circuit 5 will be described. As shown in FIG. 1, the image processing apparatus 5 includes a communication circuit 51, a reference image storage portion 52, an image scan region storage portion 53, a cine memory 54, a tomographic-image-for-saving storage portion 55, a three-dimensional guide image creation circuit 65, a mixing circuit 67, a display circuit 68, and a control circuit 60.

The control circuit 60 as a control portion includes an arithmetic device, a storage device, an input/output device, and the like, and controls the action of the image processing apparatus 5. The control circuit 60 is electrically connected to the potions or circuits in the image processing apparatus 5 via signal lines not shown. In addition, in the present embodiment, the control circuit 60 also controls the actions of the ultrasound observation apparatus 3 and the position/orientation calculation apparatus 4.

The control circuit 60 is electrically connected with the input apparatus 11 as an instruction portion. The input apparatus 11 of the present embodiment is composed of a keyboard 12 and a mouse 13. The control circuit 60 controls the action of the medical guiding system 1 based on an instruction from an operator inputted through the input apparatus 11. In the present embodiment, the keyboard 12 is provided with a group of keys including a tomographic image one-sheet saving key 12a, a tomographic image successive saving key 12b, and a scan start/termination key 12c.

Note that the input apparatus 11 is configured of the keyboard 12 and the mouse 13 in the present embodiment. However, this is one example of the input apparatus, and the input apparatus 11 is not limited to the configuration in the present embodiment. The input apparatus 11 may be configured of a pointing device such as a trackball and a touch panel, or a switch such as a push switch and rotary switch, for example. Furthermore, the input apparatus 11 may be such one that the operator can input an operation instruction by voice or gesture.

The reference image storage portion 52 is configured for example of a flash memory, a hard disk drive, a tape drive, and the like that are capable of storing a large volume of data. The reference image storage portion 52 stores a group of reference image data GRD as information on anatomical three-dimensional image of a human body.

The group of reference image data GRD is acquired such that the data of human body tomographic image acquired from a predetermined human body by the X-ray three-dimensional helical CT apparatus 16, the three-dimensional MRI apparatus 15, or the like, and stored in an external storage apparatus 17 is inputted to the image processing apparatus 5 via the network 14. As shown in FIG. 3, the group of reference image data GRD is data composed of collection of tomographic images RD of a square region with a side of several tens of centimeters of the cross section which is essentially perpendicular to the body axis (linear axis passing through from the head to the feet). The tomographic images RD are acquired at a pitch of 0.5 millimeters to several millimeters along the body axis, for example. Here, in the group of reference image data GRD shown in FIG. 3, the lower direction when facing the figure is the dorsal direction of the human body, and the depth direction from the front of the figure is the head direction of the human body.

Note that the group of reference image data GRD may be acquired from the subject 100 himself/herself, or from a different one or a plurality of human bodies. In addition, the group of reference image data GRD may be acquired from different human bodies in advance for each condition such as physical features of the human body including body height or body size, and sex. In addition, the group of reference image data GRD may be directly inputted to the image processing apparatus 5 from the X-ray three-dimensional helical CT apparatus 16 or the three-dimensional MRI apparatus 15 via the network 14 to be stored in the reference image storage portion 52.

Furthermore, as shown in FIG. 3, orthogonal coordinate axes O'-x'y'z' fixed with respect to the group of reference image data GRD are defined. Here, an origin O' is defined at lower left of the reference image data RD closest to the feet, and the x' axis and the y' axis are defined to be parallel to the lateral side and the longitudinal side, respectively. That is, the z' axis is parallel to the body axis.

In the present embodiment, as one example, the group of reference image data GRD is configured of a plurality of tomographic images RD of the abdominal region of the human body that are acquired by the X-ray three-dimensional helical CT apparatus 16. Under the effect of X-ray contrast agent, blood vessels 111 such as an aorta are displayed at a high luminance, organs such as a pancreas 110 which contain a large number of peripheral arteries are displayed at a medium luminance, and a duodenum 112 and the like are displayed at a low luminance.

The three-dimensional guide image creation circuit 65 includes an arithmetic device, an extraction circuit, a volume memory, and the like, for fast three-dimensional image processing.

The volume memory is capable of storing a large volume of data. A voxel space is assigned to at least a part of the storage region of the volume memory. The voxel space is composed of memory cells (hereinafter referred to as voxels) having addresses corresponding to the orthogonal coordinate axes O'-x'y'z' set for the group of reference image data GRD, as schematically shown in FIG. 4.

The extraction circuit extracts data of an organ of interest as a predetermined organ of a human body from the group of reference image data GRD stored in the reference image storage portion 52 to write the data in the voxel space in the volume memory as extracted data. The extracted data configures the three-dimensional model image of the organ of interest in the voxel space.

In addition, though detailed description will be made later, the three-dimensional guide image creation circuit 65 calculates index data, as three-dimensional image data expressing in the voxel space the position and orientation of the image scan range R of the ultrasound tomographic image data in the real space, by a known coordinate transformation and the like based on the position/orientation data and the like, to further create synthetic data by synthesizing the index data and the extracted data.

The index data is image data configured of at least one of an ultrasound tomographic image marker indicating the image scan range R of the latest ultrasound tomographic image data on the orthogonal coordinate axes O'-x'y'z' and an image scan history marker indicating a region where an ultrasound tomographic image data has been acquired on the orthogonal coordinate axes O'-x'y'z' in the past.

Then, the three-dimensional guide image creation circuit 65 reads out synthetic data from the voxel space in the volume memory to executes a known three-dimensional image processing such as hidden surface removal, shading, and coordinate transformation for changing the viewpoint, and creates the three-dimensional guide image data.

The mixing circuit 67 generates mixed data by synthesizing the ultrasound tomographic image data outputted from the ultrasound observation apparatus 3 and the three-dimensional guide image data outputted from the three-dimensional guide image creation circuit 65.

The display circuit 68 converts the mixed data outputted from the mixing circuit 67 into a video signal to output the video signal to the display apparatus 8. In addition, the display circuit 68 also has a configuration to convert the optical image data outputted from the optical observation apparatus 10 into a video signal and output the video signal to the display apparatus 8.

The display apparatus 8 includes an image display portion such as a cathode ray tube monitor, or a liquid crystal monitor, and has a configuration to display an image based on the video signal on the image display portion.

The cine memory 54 is configured of a memory serving as a high-speed readable/writable storage device. The cine memory 54 stores a series of plurality of ultrasound tomographic image data acquired by successive radial scans by the ultrasound transducer array 31, a plurality of position/orientation data calculated in synchronization with the respective acquisitions of plurality of ultrasound tomographic image data, and serial numbers attached to the respective plurality of ultrasound tomographic image data, in association with one another.

The image scan region storage portion 53 is configured of a memory serving as a high-speed readable/writable storage device.

The tomographic-image-for-saving storage portion 55 is configured for example of a flash memory, a hard disk drive, a tape drive, or the like. The tomographic-image-for-saving storage portion 55 stores the data instructed to be saved by the operator through the input apparatus 11, among the plurality of ultrasound tomographic image data and the data associated with the plurality of ultrasound tomographic image data that are stored in the cine memory 54.

Here, the saving instruction from the operator is made by the operator's depression of the tomographic image one-sheet saving key 12a or the tomographic image successive saving key 12b on the keyboard 12 during the scan by the ultrasound transducer array 31 of the ultrasound endoscope 2.

In the present embodiment, when the tomographic image one-sheet saving key 12a is depressed by the operator, one latest ultrasound tomographic image data at the time and the position/orientation data at the time of acquiring the ultrasound tomographic image data are read out from the cine memory 54 to be stored in the tomographic-image-for-saving storage portion 55 as tomographic-image-for-saving data which is a still image.

Furthermore, when the tomographic image successive saving key 12b is depressed by the operator, all of the ultrasound tomographic image data stored in the cine memory 54 at the time when the tomographic image successive saving key 12b was depressed are read out to be stored in the tomographic-image-for-saving storage portion 55 as the tomographic-image-for-saving data which is a moving image. That is, when the tomographic image successive saving key 12b is depressed by the operator, the tomographic-image-for-saving data is the moving image configured of a plurality of ultrasound tomographic image data successively acquired at predetermined time intervals. At the same time, the position/orientation data associated with all of the ultrasound tomographic image data stored in the cine memory 54 are also stored in the tomographic-image-for-saving storage portion 55.

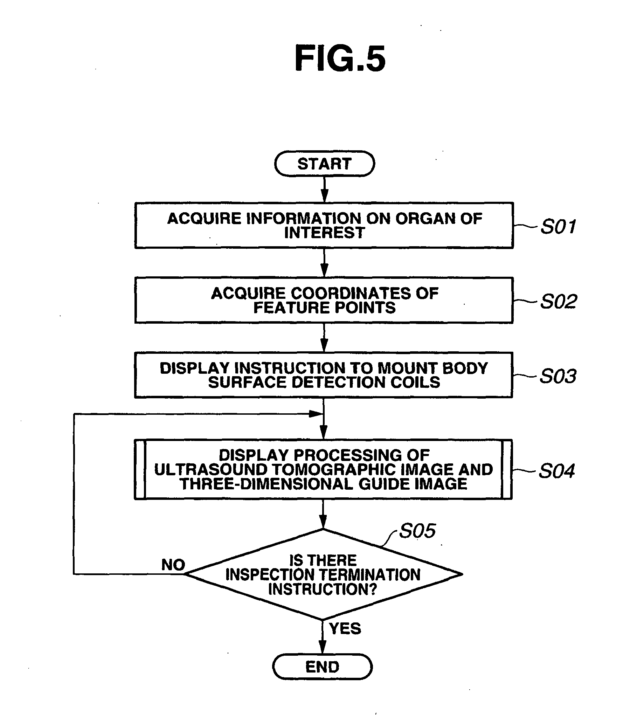

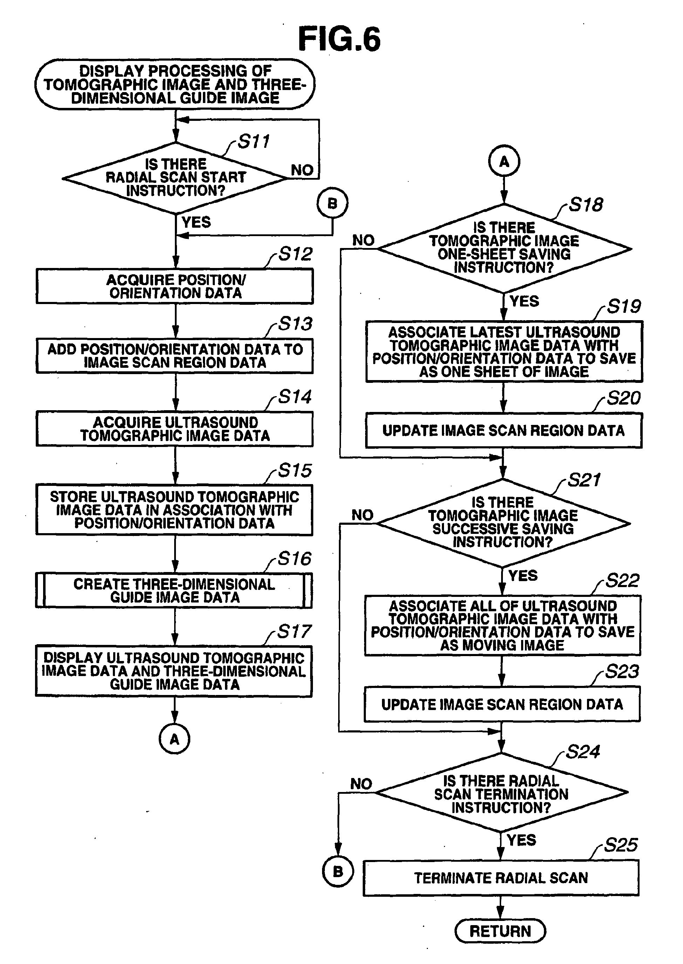

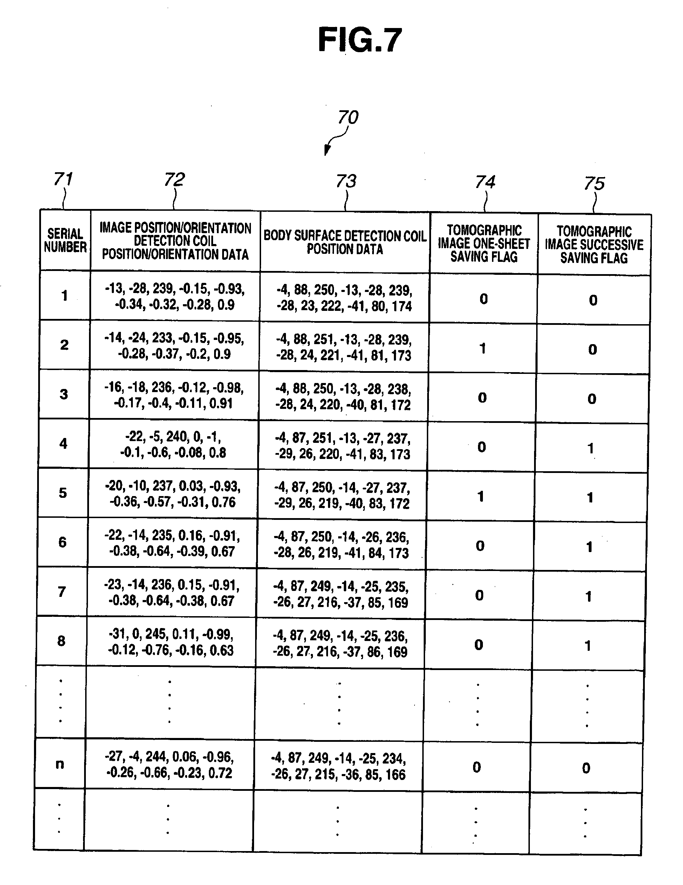

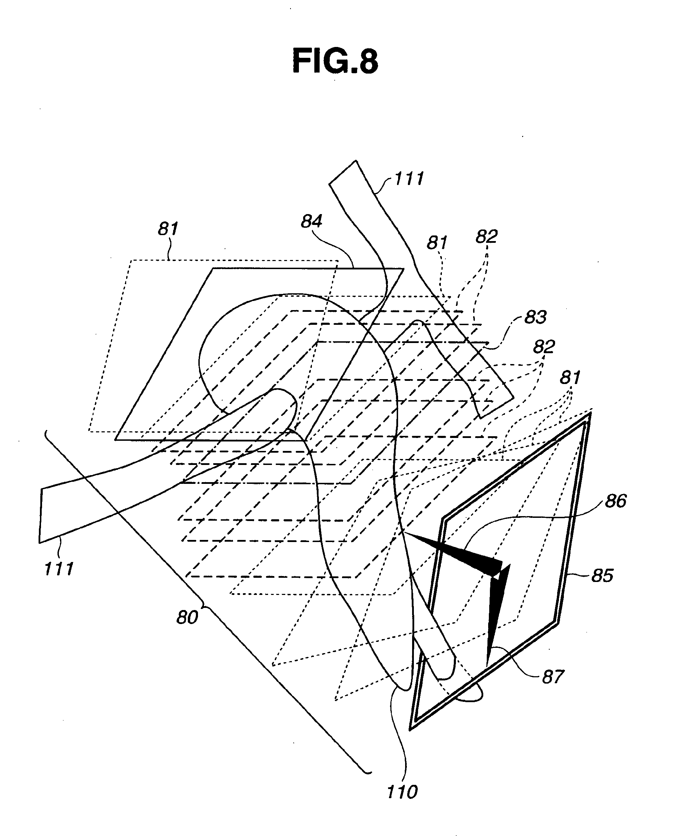

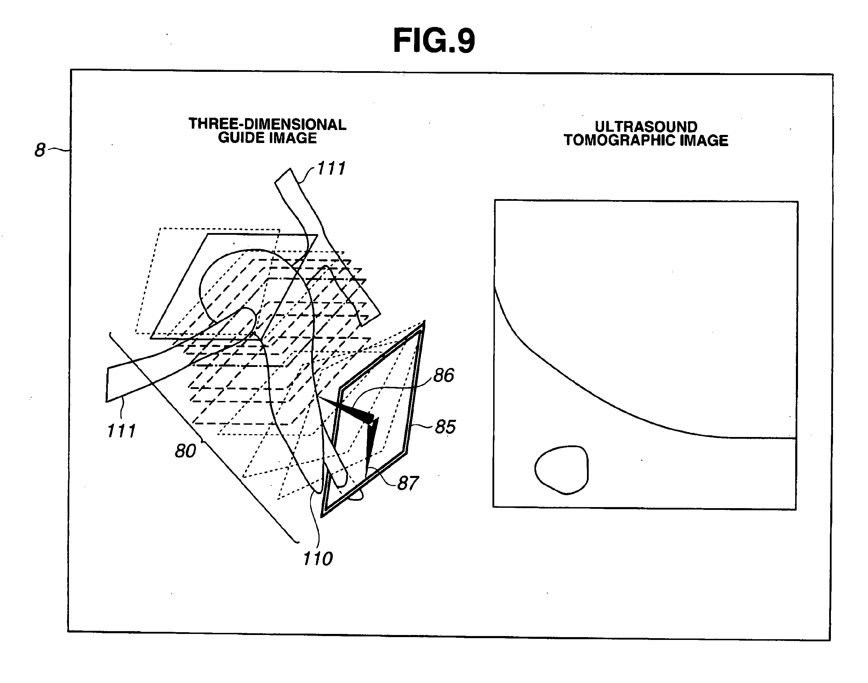

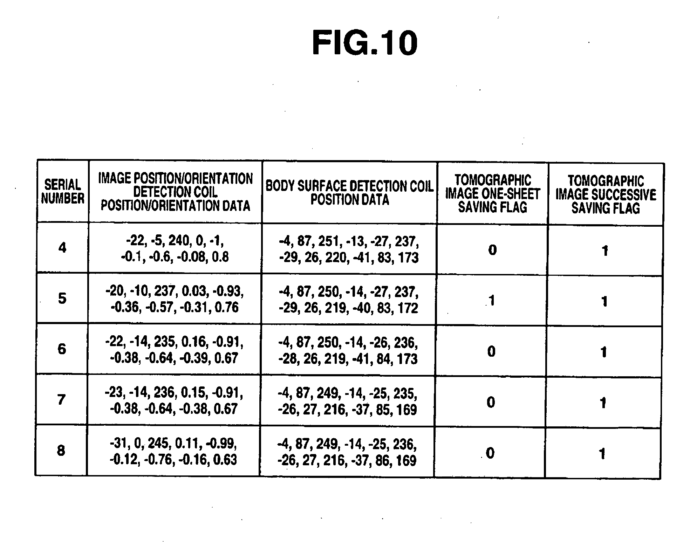

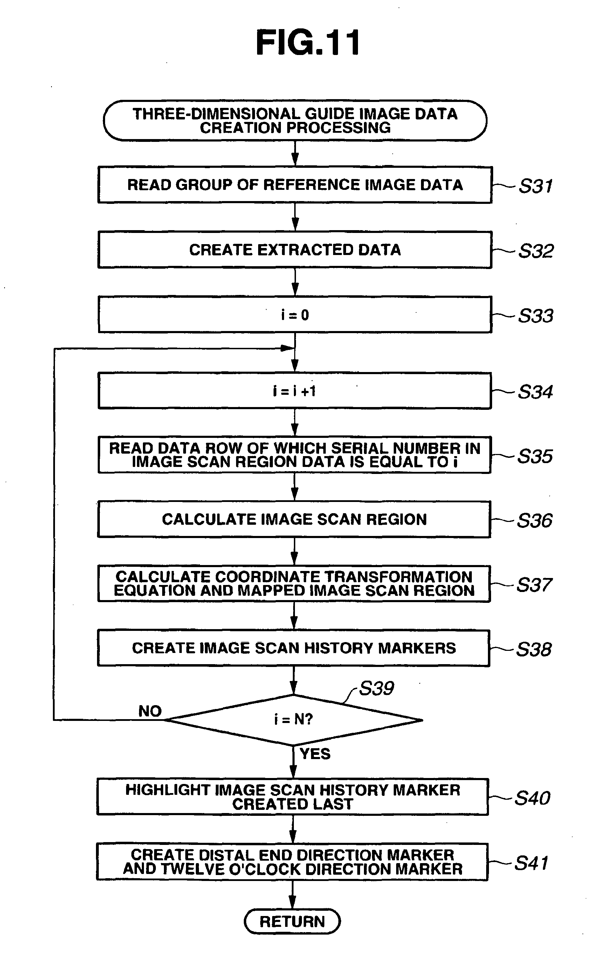

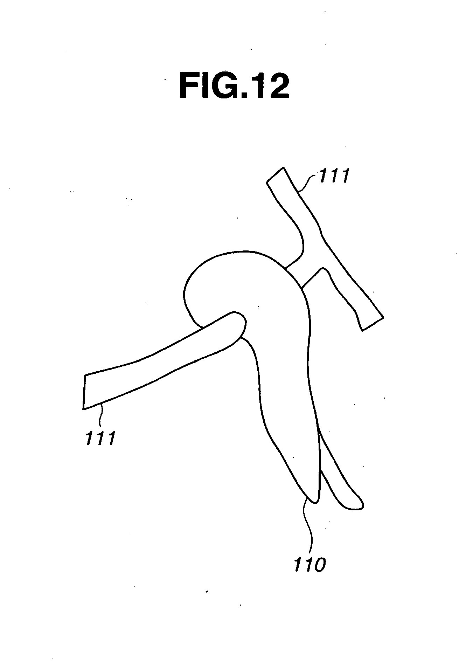

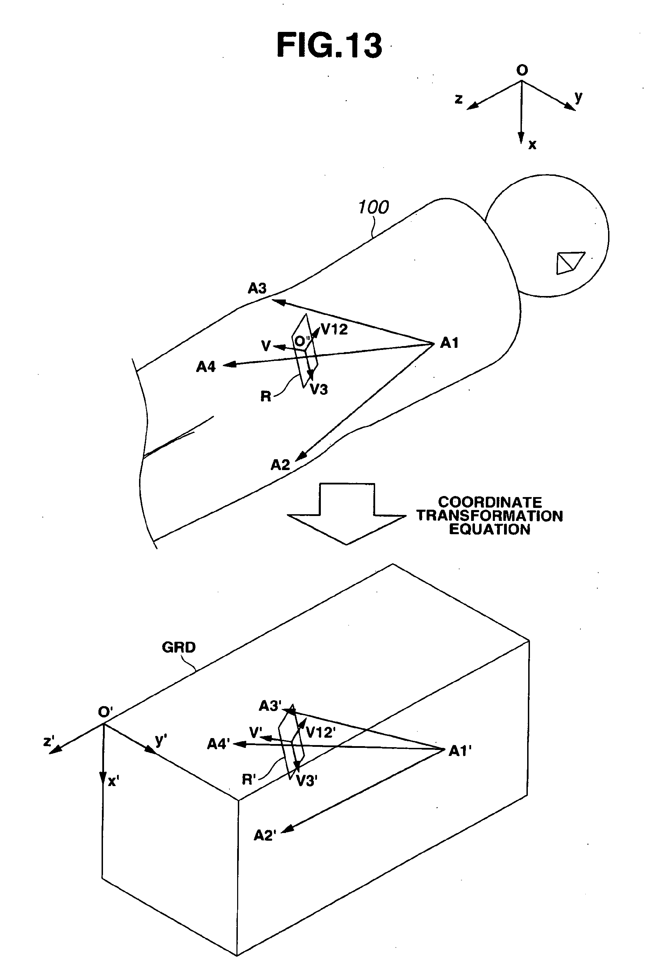

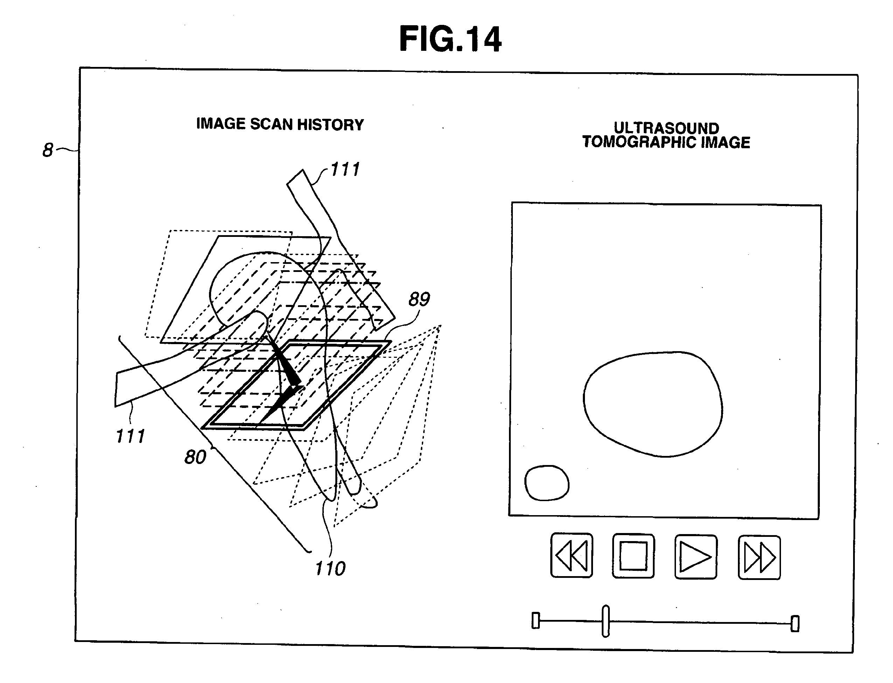

Next, the action of the medical guiding system 1 including the above configuration according to the present embodiment at the time of diagnosis is described according to an actual usage pattern by the operator. FIG 5 is a flowchart of a main routine showing an entire action of the medical guiding system. FIG. 6 is a flowchart showing a display processing of an ultrasound tomographic image and a three-dimensional guide image. FIG. 7 is a table showing one example of image scan region data. FIG. 8 is a view describing a three-dimensional guide image. FIG. 9 is a view describing mixed data displayed on a display apparatus. FIG 10 is a table showing one example of files saved in a tomographic-image-for-saving storage portion. FIG. 11 is a flowchart showing a creation processing of three-dimensional guide image data. FIG. 12 is one example of a three-dimensional model image of an organ of interest. FIG. 13 is a view describing the concept of coordinate transformation. FIG. 14 is a view describing an image scan history reproduced image.

Note that description will be made hereinafter taking as an example a case where a diagnosis is performed by inserting the rigid portion 21 and the flexible portion 22 of the ultrasound endoscope 2 into the body of the subject 100 and the pancreas is extracted and displayed as the organ of interest at the time of performing diagnosis.

First, the control circuit 60 acquires information on the organ of interest indicating the kind of the organ to be diagnosed (step S01). Here, the control circuit 60 displays options on the display apparatus 8 to prompt the operator to select an organ of the human body. Then, the operator specifies one or a plurality of organs of interest from the options of the organs displayed on the display apparatus 8 through the input apparatus 11, and thereby the information on the organ of interest is inputted to the control circuit 60. In the present embodiment, the pancreas is specified as the organ of interest as described above.

Note that the selection screen of the organs of interest which is displayed on the display apparatus 8 may display the information on the organs of the human body by a two-dimensional or three-dimensional image on which the operator visually selects the organ of interest. Alternatively, the organs of the human body may be displayed as character strings from which the operator selects the organ of interest.

Next, the control circuit 60 sets a plurality of feature points A' in the group of reference image data GRD by an input of instruction by the operator or an automatic calculation, to execute a processing of acquiring the coordinates on the orthogonal coordinate axes O'-x'y'z' of the plurality of feature points A' (step S02). The plurality of feature points A' in the group of reference image data GRD anatomically correspond to the plurality of sample points A on the body surface or inside the body of the subject 100.

In the present embodiment, the feature points A' are the feature points A1' to A4' respectively corresponding to the sample points A1 to A4. That is, the feature points A1', A2', A3' and A4' are pixels in the group of reference image data GRD corresponding to the xiphoid process, the left anterior superior iliac spine, the right anterior superior iliac spine, and the spinous process of vertebral body, respectively.

In the step S02, the pixels corresponding to the plurality of feature points A1' to A4' in the group of reference image data GRD are specified by the input from the operator through the input apparatus 11, for example, thereby acquiring and storing the coordinates on the orthogonal coordinate axes O'-x'y'z' of the respective plurality of feature points A1' to A4'.

Next, the control circuit 60 provides a display to prompt the operator to mount the body surface detection coils 42 on the subject 100 on the display apparatus 8 (step S03). The operator mounts and fixes the body surface detection coils 42 in the vicinity of sample points on the body surface of the subject 100.

In the present embodiment, the four body surface detection coils 42 are respectively mounted by the operator in the vicinity of the sample point A 1 (xiphoid process), the sample point A2 (left anterior superior iliac spine), the sample point A3 (right anterior superior iliac spine), and the sample point A4 (spinous process of vertebral body) of the subject 100.

Next, the control circuit 60 executes a display processing, to be described in detail later, of the ultrasound tomographic image and the guide image (step S04). In the step S04, the operator inserts the rigid portion 21 and the flexible portion 22 of the ultrasound endoscope 2 into the body of the subject 100 and makes diagnosis while referring to the ultrasound tomographic image data and the three-dimensional guide image data displayed on the display apparatus 8.

The control circuit 60 repeatedly executes the processing in the step S04 until an inspection termination key not shown on the keyboard 12 is depressed by the operator (step S05). In the step S05, when determining that the inspection termination key has been depressed by the operator, the control circuit 60 associates image scan region data 70 stored in the image scan region storage portion 53 with the ultrasound tomographic image data stored in the tomographic-image-for-saving storage portion 55, to execute termination processing.

Next, with reference to the flowchart in FIG. 6, description is made in detail on the display processing of the ultrasound tomographic image and the three-dimensional guide image in the step S04 in the flowchart shown in FIG. 5.

First, the control circuit 60 stands by until the operator depresses the scan start/termination key 12c on the keyboard 12 to give an instruction to start radial scan (step S11). When determining that the scan start/termination key 12c on the keyboard 12 was depressed and instruction to start the radial scan was inputted, the control circuit 60 executes an initial processing for starting radial scan by the ultrasound transducer array 31, and the procedure moves on to the step S12.

Next, the three-dimensional guide image creation circuit 65 acquires the position/orientation data outputted from the position/orientation calculation apparatus 4 (step S12). The position/orientation data is configured of the data of the position O" and the orientation of the image position/orientation detection coil 44 and the data of the respective positions of the four body surface detection coils 42, on the orthogonal coordinate axes O-xyz.

Next, the three-dimensional guide image creation circuit 65 assigns a serial number to the position/orientation data acquired in the step S12 to store the serial number and the position/orientation data in the image scan region storage portion 53 as the image scan region data 70 (step S13). The image scan region data 70 is matrix-shaped data as shown in the table in FIG 7.

The column direction of the image scan region data 70 is configured of five kinds of data, that is, "serial number 71 ", "image position/orientation detection coil position/orientation data 72", "body surface detection coil position data 73", "tomographic image one-sheet saving flag 74", and "tomographic image successive saving flag 75".

In the image scan region data 70, every time the step S 13 is executed, the latest data regarding the kinds of data in the column direction are sequentially added in the row direction.

The serial number 71 is a natural number not less than 1, and is data such that the first value of which immediately after the start instruction of the radial scan is set to 1, and thereafter every time the step S 13 is executed, the value is incremented by one, like 2, 3, 4, etc. That is, the serial number 71 is a value indicating the data acquisition order.

As described above, the image position/orientation detection coil position/orientation data 72 is the data in which the values of coordinates and coordinate components on the orthogonal coordinate axes O-xyz are described in the following order: the x-, y- and z-coordinate values of the position O" of the image position/orientation detection coil 44; the x-, y- and z-coordinate components of the base vector V; and the x-, y- and z-coordinate components of the base vector V3.

The body surface detection coil position data 73 is the data in which the respective x-, y- and z-coordinate values on the orthogonal coordinate axes O-xyz of the four body surface detection coils 42 are described. In the present embodiment, the respective x-, y- and z-coordinate values of the body surface detection coils 42 mounted on the sample points A1 to A4 of the subject 100 are described in the order of the sample points A1, A2, A3, and A4.

Though details will be described later, the tomographic image one-sheet saving flag 74 and the tomographic image successive saving flag 75 are binary data of 0 or 1, and 0 is written when newly described.

Specifically, in the step S 13, the three-dimensional guide image creation circuit 65 first reads the largest value of the serial numbers 71 in the image scan region data 70. Then the three-dimensional image creation circuit 65 creates and adds a data row having a new serial number acquired by adding one to the largest serial number 71, and then further writes the latest image position/orientation detection coil position/orientation data 72 and the body surface detection coil position data 73 that are acquired in the immediately preceding step S12, into the data row having the new serial number. In addition, the three-dimensional image creation circuit 65 writes the value 0 as the respective values of the tomographic image one-sheet saving flag 74 and the tomographic image successive saving flag 75, into the data row having the new serial number.

Note that, if the image scan region data 70 is not stored in the image scan region storage portion 53 in the step S 13, the three-dimensional guide image creation circuit 65 newly creates a file of the image scan region data 70 in which to write a data row, the serial number 71 of which is 1. Then, the three-dimensional guide image creation circuit 65 writes the latest image position/orientation detection coil position/orientation data 72 and the body surface detection coil position data 73, which are acquired in the immediate preceding step S12, into the data row, the serial number 71 of which is 1. Furthermore, the three-dimensional guide image creation circuit 65 writes the value 0 as the respective values of the tomographic image one-sheet saving flag 74 and the tomographic image successive saving flag 75, into the data row, the serial number of which is 1.

Next, the mixing circuit 67 acquires the ultrasound tomographic image data outputted from the ultrasound observation apparatus 3 (step S 14). The ultrasound tomographic image data is acquired synchronously with the position/orientation data.

Next, the control circuit 60 stores in the cine memory 54 the ultrasound tomographic image data acquired by the mixing circuit 67 in the step S 14, the position/orientation data acquired in the step S12, and the serial number assigned to the position/orientation data in step S 13, in association with one another (step S 15).

That is, every time the step S 15 is executed, four kinds of data, i.e., the ultrasound tomographic image data, the image position/orientation detection coil position/orientation data, the body surface detection coil position data, and the serial number are stored in association with one another as one set of data, in the cine memory 54.

In the step S 15, when no storage capacity for storing new set of data is left in the cine memory 54, the above-described new set of data is stored after one set of data with the smallest serial number is deleted.

Next, the three-dimensional image creation circuit 65 creates the three-dimensional guide image data as shown in FIG. 8 by a method to be described later in detail, to output the created three-dimensional guide image data to the mixing circuit 67 (step S16). Roughly describing, the three-dimensional guide image data is three-dimensional image data indicating the positions of the image scan range R of the past and the latest ultrasound tomographic image data with respect to an organ of interest. Note that the viewpoint of the three-dimensional guide image data as the three-dimensional image data can be set at an arbitrary position by an instruction inputted by the operator.

Next, the mixing circuit 67 creates mixed data for display as shown in FIG. 9 by synthesizing the ultrasound tomographic image data acquired in the step S 14 and the three-dimensional guide image data inputted in the step S16. Then, the display circuit 68 converts the mixed data created by the mixing circuit 67 into a video signal to output the video signal to the display apparatus 8. The display apparatus 8 adjacently displays the ultrasound tomographic image data and the three-dimensional guide image data, based on the video signal (step S 17).

Next, the control circuit 60 determines whether or not the tomographic image one-sheet saving key 12a on the keyboard 12 has been depressed by the operator during the execution of the steps S12 to S 17 (step S 18).

In the step S 18, when the control circuit 60 determines that the tomographic image one-sheet saving key 12a has been depressed, the procedure moves on to step S 19. On the other hand, in the step S 18, when the control circuit 60 determines that the tomographic image one-sheet saving key 12a is not depressed, the procedure moves on to step S21.

In the step S 19, the control circuit 60 stores in the tomographic-image-for-saving storage portion 55 the latest data stored in the cine memory 54, i.e, the latest ultrasound tomographic image data, image position/orientation detection coil position/orientation data, and body surface detection coil position data, in association with one another.

Next, in the step S20, the control circuit 60 rewrites to 1 the value of the tomographic image one-sheet saving flag 74 in the latest data row in the image scan region data 70 stored in the image scan region storage portion 53, to update the image scan region data 70. In other words, in the step S20, rewriting is performed on the data row in the image scan region data 70, including the value of the serial number corresponding to the ultrasound tomographic image data saved in the tomographic-image-for-saving storage portion 55 in the step S19. Then the procedure moves on to the step S21.

In the step S21, the control circuit 60 determines whether or not the tomographic image successive saving key 12b on the keyboard 12 has been depressed by the operator during the execution of the steps S12 to S 17.

In the step S21, when the control circuit 60 determines that the tomographic image successive saving key 12b has been depressed, the procedure moves on to step S22. On the other hand, in the step S21, when the control circuit 60 determines that the tomographic image successive saving key 12b is not depressed, the procedure moves on to step S24.

In the step S22, the control circuit 60 stores in the tomographic-image-for-saving storage portion 55 all of the data stored in the cine memory 54, i.e, all of the ultrasound tomographic image data, the image position/orientation detection coil position/orientation data, and the body surface detection coil position data, in association with one another.

For example, at the time of execution of the processing in step S22, when data of five sheets of ultrasound tomographic images corresponding to the serial number values 4 to 8 and the position/orientation data corresponding to the five sheets of the ultrasound tomographic images are stored in the cine memory 54, all of the data are saved in the tomographic-image-for-saving storage portion 55 by the processing of the step S22.

In this case, specifically, together with data of the five sheets of ultrasound tomographic images, one file as shown in FIG. 10, in which the serial numbers and the position/orientation data corresponding to the five sheets of ultrasound tomographic images are described, is saved in the tomographic-image-for-saving storage portion 55. In addition, the data of the five sheets of ultrasound tomographic images are saved with the same file names as the corresponding serial numbers. That is, the ultrasound tomographic image data are associated with the serial numbers and the position/orientation data by the file names.

Next, in the step S23, the control circuit 60 rewrites to 1 the value of the tomographic image successive saving flag 75 in the data row corresponding to the ultrasound tomographic image saved in the step S22 in the image scan region data 70 stored in the image scan region storage portion 53, to update the image scan region data 70. In other words, in the step S23, rewriting is performed on the data row in the image scan region data 70, the data row including the value of the serial number corresponding to the ultrasound tomographic image data saved in the tomographic-image-for-saving storage portion 55 in the step S22. Then the processing moves on to the step S24.

Next, in the step S24, the control circuit 60 determines whether or not the scan start/termination key 12c on the keyboard 12 has been depressed by the operator during the execution of the steps S12 to S23.

In the step S24, when the control circuit 60 determines that the scan start/termination key 12c has not been depressed in the step S24, the procedure returns to the step S12 and the above-described processings are repeated.

Thus, the medical guiding system 1 of the present embodiment repeatedly executes the steps S12 to S23 at a predetermined cycle until the scan start/termination key 12c is depressed again, thereby sequentially creating new three-dimensional guide image data, to display in real time the created three-dimensional guide image data together with new ultrasound tomographic image data on the image display portion of the display apparatus 8.

On the other hand, in the step S24, when the control circuit 60 determines that the scan start/termination key 12c has been depressed, the procedure moves on to the step S25, and the control circuit 60 outputs a signal for stopping the radial scan by the ultrasound transducer array 31 to the ultrasound observation apparatus 3 to terminate the radial scan, thereby terminating the display processing of the ultrasound tomographic image and the three-dimensional guide image.

Next, with reference to the flowchart in FIG. 11, description will be made on the detail of the creation processing of the three-dimensional guide image data in the step S16 in the flowchart shown in FIG. 6.

First, the three-dimensional image creation circuit 65 reads the group of reference image data GRD stored in the reference image storage portion 52 (step S31).

Next, the three-dimensional image creation circuit 65 extracts the data of the organ of interest from the group of reference image data GRD to write the data in the voxel space in the volume memory as extracted data (step S32). The three-dimensional image creation circuit 65 creates the three-dimensional model image displaying only the pancreas 110 as the organ of interest and the blood vessels 111 as shown in FIG. 12 for example, as extracted data, and writes the extracted data in the voxel space.

Next, the three-dimensional guide image creation circuit 65 defines a variable i to be stored therein, and sets the value of the variable I to 0 (step S33).

Next, the three-dimensional guide image creation circuit 65 adds 1 to the variable i stored therein (step S34). That is, the three-dimensional guide image creation circuit 65 performs a calculation, i = i + 1.

Next, the three-dimensional guide image creation circuit 65 reads the data row, the value of the serial number 71 of which is equal to the variable i, from the image scan region data 70 stored in the image scan region storage portion 53 (step S35).

Then, the three-dimensional guide image creation circuit 65 calculates the image scan range R of the ultrasound tomographic image data on the orthogonal coordinate axes O-xyz as a real space from a set value of the ultrasound scanning range, that is, the set value of the length of one side of the ultrasound tomographic image data in a square shape, and the image position/orientation detection coil position/orientation data among the data read in the step S35 (step S36).

Specifically, in the step S36, the three-dimensional guide image creation circuit 65 calculates the coordinates on the orthogonal coordinate axes O-xyz, of the four vertices R1 to R4 of the image scan range R of the ultrasound tomographic image in a square shape, based on the image position/orientation detection coil position/orientation data, that is, the position O" of the image position/orientation detection coil 44 and the base vectors V and V3 indicating the orientation of the image position/orientation detection coil 44.

In the step S36, first the base vector V12 is calculated by calculating the outer product of the base vectors V and V3. Then, assuming that the length of one side of the ultrasound tomographic image data is a, the coordinates of the first vertex R1 are calculated based on the equation O" + a/2 • (V12) + a/2 • (V3), the coordinates of the second vertex R2 based on the equation O"- a/2 • (V12) + a/2 • (V3), the coordinates of the third vertex R3 based on the equation O" + a/2 • (V12) - a/2 • (V3), and the coordinates of the fourth vertex R4 based on the equation O"-a/2 • (V12) - a/2 • (V3).

Next, the three-dimensional guide image creation circuit 65 calculates a coordinate transformation equations for mapping the coordinates on the orthogonal coordinate axes O-xyz to the coordinates on the orthogonal coordinate axes O'-x'y'z', based on the body surface detection coil position data among the data read in the step S35, that is, the coordinates on the orthogonal coordinate axes O-xyz of the plurality of body surface detection coils 42, and the coordinates on the orthogonal coordinate axes O'-x'y'z' of the plurality of feature points A' in the group of reference image data GRD, which were acquired in the step S02. Then, as conceptually shown in FIG. 13, the three-dimensional guide image creation circuit 65, by using the coordinate transformation equation, maps the image scan range R of the ultrasound tomographic image data on the orthogonal coordinate axes O-xyz, which was calculated in the step S36, on the orthogonal coordinate axes O'-x'y'z' (step S37).

Hereinafter, the image scan range R mapped on the orthogonal coordinate axes O'-x'y'z' is referred to as a mapped image scan range R'. Specifically, the mapped image scan range R' is a quadrilateral planar region defined by the four points R1' to R4' acquired by mapping the four vetices R1 to R4 calculated in the step S36 on the orthogonal coordinate axes O'-x'y'z' using the coordinate transformation equations.

Next, the three-dimensional guide image creation circuit 65 creates image scan history markers 80 as the index data indicating the region corresponding to the mapped image scan range R' on the orthogonal coordinate axes O'-x'y'z' in the step S37, and writes the image scan history markers 80 in the voxel space in the volume memory to create synthetic data (step S38).

The image scan history markers 80 are three-dimensional image data each expressing a translucent quadrilateral plane having four points R1' to R4' calculated in the step S37 as vertices, as shown in FIG. 8. Here, since the extracted data of the organ of interest has already been written in the voxel space in the step S32, the synthetic data in the voxel space is created such that the image scan history markers 80 are superimposed on the extracted data of the organ of interest, as shown in FIG. 8.

Furthermore, in the step S38, the three-dimensional guide image creation circuit 65 draws the image scan history markers 80 such that the line types of the border lines indicating the perimeters of the quadrilateral image scan history markers 80 are changed depending on the values of "the tomographic image one-sheet saving flag 74" and "the tomographic image successive saving flag 75" among the data read in the step S35.

In the present embodiment, as shown in FIG. 8, when both of the values of the tomographic image one-sheet saving flag 74 and the tomographic image successive saving flag 75 are 0, the line type of the border lines is a dotted line (the reference numeral 81 in the FIG. 8), when both of the values of the tomographic image one-sheet saving flag 74 and the tomographic image successive saving flag 75 are 1, the line type of the border lines is a dashed line (the reference numeral 82 in the FIG. 8), when the value of the tomographic image one-sheet saving flag 74 is 0 and the value of the tomographic image successive saving flag 75 is 1, the line type of the border lines is a chain line (the reference numeral 83 in FIG. 8), and when the value of the tomographic image one-sheet saving flag 74 is 1 and the value of the tomographic image successive saving flag 75 is 0, the line type of the border lines is a solid line (the reference numeral 84 in FIG. 8).

Note that it is enough that the image scan history markers 80 are displayed in different ways depending on the corresponding values of the tomographic image one-sheet saving flag 74 and the tomographic image successive saving flag 75, and the markers are not limited to the aspect of display differentiated by the line types as in the present embodiment. For example, the image scan history markers 80 may be displayed in different colors depending on the corresponding values of the tomographic image one-sheet saving flag 74 and the tomographic image successive saving flag 75, or differentiated by brightness difference or cyclical blinking, for example.

Next, the three-dimensional guide image creation circuit 65 determines a magnitude relation between the largest value N of the serial number 71 in the image scan region data 70 stored in the image scan region storage portion 53 and the variable i (step S39).

As a result of the determination in step S39, when the variable i is smaller than the largest value N of the serial number 71, the procedure returns to the step S34 and the processings in steps S34 to S38 are repeated. That is, when there are plurality of data rows described in the image scan region data 70, the processings in the steps S34 to S38 are repeated on all of the data rows, and thereby a plurality of image scan history markers 80 as shown in FIG. 8 are written in the voxel space in the volume memory as index data.

On the other hand, as a result of the determination in the step S39, when the variable i is equal to the largest value N of the serial number 71, the procedure moves on to step S40.

In the step S40, the three-dimensional guide image creation circuit 65 changes the image scan history marker 80 created last, to highlight the marker as an image scan region marker 85. Here, the image scan history marker 80 created last is the image scan history marker 80 corresponding to the data row, the serial number 71 of which in the image scan region data 70 is the largest value N. In the present embodiment, the three-dimensional guide image creation circuit 65 changes the line type of the border line indicating the perimeter of the image scan region marker 85 to a thick line, to write the image scan region marker 85 in the voxel space.

In other words, the image scan region marker 85 created in the step S40 is the index indicating the mapped image scan range R' on the orthogonal coordinate axes O'-x'y'z' of the latest ultrasound tomographic image data.

Next, the three-dimensional guide image creation circuit 65 creates a distal end direction marker 86 and a twelve o'clock direction marker 87 as indexes to indicate the orientation of the mapped image scan range R' on the orthogonal coordinate axes O'-x'y'z' of the latest ultrasound tomographic image data, and writes the markers in the voxel space in the volume memory (step S41).

The distal end direction marker 86 is an arrow-shaped (pyramid-shaped) three-dimensional object which is perpendicular to the image scan region marker 85 from the barycentric position of the image scan region marker 85 and extends in the insertion direction of the rigid portion 21 on the orthogonal coordinate axes O'-x'y'z'. Furthermore, the twelve o'clock direction marker 87 is an arrow-shaped image to indicate the twelve o'clock direction of the radial scan on the orthogonal coordinate axes O'-x'y'z', which is drawn on the same plane as the image scan region marker 85.

Specifically, in the step S41, the three-dimensional guide image creation circuit 65 calculates a coordinate transformation equation for mapping the coordinates on the orthogonal coordinate axes O-xyz to the coordinates on the orthogonal coordinate axes O'-x'y'z', based on the body surface detection coil position data in the data row, the serial number 71 of which in the image scan region data 70 is the largest value N, and the coordinates on the orthogonal coordinate axes O'-x'y'z' of the plurality of feature points A' in the group of reference image data GRD.

Then, the three-dimensional guide image creation circuit 65, by using the coordinate transformation equation, calculates the base vectors V' and V3' by mapping the base vectors V and V3 on the orthogonal coordinate axes O'-x'y'z', in the data row, the serial number 71 of which in the image scan region data 70 is the largest value N. Then, the three-dimensional guide image creation circuit 65 calculates the base vector V 12' by calculating the outer product of the base vectors V and V3.

Then, the three-dimensional guide image creation circuit 65 creates the distal end direction marker 86 and the twelve o'clock direction marker 87 which are the arrow-shaped image data indicating the directions of the base vectors V' and V12' on the orthogonal coordinate axes O'-x'y'z', respectively, to write the markers in the voxel space in the volume memory.

Note that the image scan region marker 85, the distal end direction marker 86, and the twelve o'clock direction marker 87 configure the ultrasound tomographic image marker indicating the image scan range R of the latest ultrasound tomographic image data on the orthogonal coordinate axes O'-x'y'z' in the present embodiment.

By executing the processings from the steps S31 to S41, the three-dimensional guide image creation circuit 65 creates three-dimensional guide image data.

Next, description will be made on the action of the medical guiding system 1 of the present embodiment at the time of reproducing the image scan history.

The medical guiding system of the present embodiment 1 is capable of performing not only an action of displaying by changing in real time the current image scan position and the history of the past image scan position of the ultrasound tomographic image with respect to the organ of the subject as the three-dimensional guide images, at the time of making diagnosis with the above-described ultrasound diagnostic apparatus, but also an action of reproducing and displaying the history of the image scan position of the ultrasound tomographic image so that the operator can perform observation while confirming the ultrasound tomographic image and the position where the ultrasound tomographic image was scanned at the same time, after the diagnosis.

When an image scan history reproducing key, not shown, on the keyboard 12 is depressed by the operator, the image processing apparatus 5 as an ultrasound tomographic image reproducing portion creates an image scan history reproduced image as shown in FIG. 14, to display the created image scan history reproduced image on the image display portion of the display apparatus 8.

The image scan history reproduced image is an image in which the ultrasound tomographic image data is displayed on the right side of the image display portion of the display apparatus 8 and the position of the currently displayed ultrasound tomographic image data with respect to the organ of interest is displayed on the left side, in the present embodiment.

Specifically, when the image scan history reproducing key on the keyboard 12 is depressed by the operator, the control circuit 60 reads a predetermined one sheet of ultrasound tomographic image data specified by the operator through the input apparatus 11 among all of the ultrasound tomographic image data stored in the tomographic-image-for-saving storage portion 55.

In addition, when the image scan history reproducing key on the keyboard 12 is depressed by the operator, the three-dimensional guide image creation circuit 65 executes similar processings as those in the above-described steps S31 to S39, to create image scan history image data on which the three-dimensional model image of the organ of interest and a plurality of image scan history markers 80 based on all of the data rows recorded in the image scan region data 70 stored in the image scan region storage portion 53 are superimposed on each other. That is, the image scan history markers 80 are three-dimensional image data indicating the positions and the orientations of the image scan regions of all of the ultrasound tomographic image data acquired during the diagnosis.

Then, the three-dimensional guide image creation circuit 65 changes the line type of the border line indicating the perimeter of the image scan history marker 80 corresponding to the predetermined one sheet of the ultrasound tomographic image data specified by the operator, among the plurality of image scan history markers 80, to a thick line to highlight the image scan history marker (the reference numeral 89 in FIG. 14). Furthermore, the three-dimensional guide image creation circuit 65 creates the distal end direction marker 86 and the twelve o'clock direction marker 87 that indicate the orientation of the predetermined one sheet of the ultrasound tomographic image data specified by the operator.

Next, the mixing circuit 67 generates mixed data in which the predetermined one sheet of ultrasound tomographic image data read by the control circuit 60 and the image scan history image data created by the three-dimensional guide image creation circuit 65 are synthesized as shown in FIG. 14 and outputs the generated mixed data. Then, the display apparatus 8 displays the image scan history reproduced image based on the mixed data on the image display portion.

In the present embodiment, when an instruction to switch the currently displayed ultrasound tomographic image data to another ultrasound tomographic image data is inputted by the operator through the input apparatus 11 in a state where the image scan history reproduced image is displayed, the three-dimensional guide image creation circuit 65 changes the image scan history marker 80 to be highlighted and to which the distal end direction marker 86 and the twelve o'clock direction marker 87 are to be added, to the one corresponding to the ultrasound tomographic image data to be newly displayed by the switching.

For example, in the medical guiding system 1 of the present embodiment, every time the operator depresses a predetermined key on the keyboard 12 in a state where the image scan history reproduced image is displayed, the ultrasound tomographic image data are sequentially switched and displayed on the image display portion of the display apparatus 8 in the order of the times when the data were saved during inspection.

Then, according to the switching of the ultrasound tomographic image data to be displayed, the image scan history marker 80 to be highlighted and to which the distal end direction marker 86 and the twelve o'clock direction marker 87 are to be added in the image scan history reproduced image is also switched and displayed. Here, if the currently displayed ultrasound tomographic image data is a part of a moving image by successive saving, the moving image is reproduced and displayed on the image display portion of the display apparatus 8 when a reproducing key, not shown, is depressed by the operator.

Note that, the aspect of the image scan history markers 80 displayed in the image scan history reproduced image is not limited to one in which all of the image scan history markers 80 corresponding to all of the ultrasound tomographic image data acquired during inspection are displayed, but the image scan history marker 80 corresponding only to the data row in which at least one of the values of the tomographic image one-sheet saving flag 74 and the tomographic image successive saving flag 75 in the image scan region data 70 stored in the image scan region storage portion 53 is 1, for example.

Thus, by selectively displaying the image scan history marker 80, the load of processings required for display can be reduced. In addition, visibility of the image scan history marker 80 is improved, thereby enabling the operator to easily grasp the positional relationship between the organ of interest and the image scan history marker 80.

Effects of the above-described medical guiding system 1 of the present embodiment are described below.

In the medical guiding system 1 of the present embodiment, during the ultrasound scan by the ultrasound endoscope 2, the image scan history markers 80 indicating a history of the regions where the ultrasound tomographic image data were acquired in the past are displayed superimposed on the three-dimensional model image of the organ of interest.

Therefore, using the medical guiding system 1 of the present embodiment can make it easier for the operator to visually recognize the positional relationship of the past image scan regions with respect to the organ of interest, that is, the history of the regions where ultrasound diagnosis has been already made with respect to the organ of interest. Therefore, the operator can surely make an ultrasound diagnosis with respect to the organ of interest without oversight.

In addition, using the medical guiding system 1 of the present embodiment can make it easier for the operator to visually recognize the history of the regions where ultrasound diagnosis has already been made with respect to the organ of interest, thereby eliminating repeated scan on the same region and shortening the diagnosis time. As a result, the burdens on the operator and the subject are reduced.

In addition, in the medical guiding system 1 of the present embodiment, during the ultrasound scan by the ultrasound endoscope 2, the image scan positions of the tomographic-image-for-saving data are also displayed superimposed on the three-dimensional model image of the organ of interest as the image scan history markers 80.

Therefore, using the medical guiding system 1 of the present embodiment can make it easier for the operator to visually check the image scan positions of the saved ultrasound tomographic image data with respect to the organ of interest. As a result, the operator can surely remember to save the ultrasound tomographic image data required for diagnosis.

Furthermore, in the medical guiding system 1 of the present embodiment, the regions where the ultrasound diagnosis has been performed, the tomographic-image-for-saving data, and the image scan positions of the tomographic-image-for-saving data are saved, and reproduced and displayed as the image scan history reproduced images after diagnosis. Therefore, the operator confirms these data as the image scan history reproduced images after diagnosis, thereby enabling the work such as sorting the medical records to be performed more easily and in a shorter time.

In addition, in the medical guiding system 1 in the present embodiment, the regions where the ultrasound diagnosis has been performed, the tomographic-image-for-saving data, and the image scan positions of the tomographic-image-for-saving data are saved, and reproduced and displayed as the image scan history reproduced images after diagnosis. Therefore, by using the medical guiding system 1 of the present embodiment, the operator can confirm the image scan history reproduced images after diagnosis to compare the ultrasound tomographic image data with the image scan positions of the ultrasound tomographic data with respect to the organ of interest, thereby easily confirming a position of a legion such as a tumor three-dimensionally and drawing up a precise operation plan.

Note that, in the above-described present embodiment, the position/orientation data and the ultrasound tomographic image data are associated with each other by the serial numbers and the file names. However, the method of associating the data is not limited to this. For example, the position/orientation data and the ultrasound tomographic image data may be associated with each other by data of the time when each of the data was acquired and generated.

In addition, in the present embodiment, the length a of one side of the image scan range R of the ultrasound tomographic image data is assumed to be a fixed value. That is, the shape and area of the image scan range R are assumed to be fixed. However, the present invention is not limited to this aspect. For example, if the shape and area of the image scan range R of the ultrasound tomographic image data are variable by changing the intensity and the scan range of the ultrasound beam transmitted by the ultrasound transducer array 31, also the scan range information is stored as the image scan region data 70 in association with the ultrasound tomographic image data, and the image scan history marker 80 is displayed in a size and shape corresponding to the change in the intensity and the scan range of the ultrasound beam.

Furthermore, in the present embodiment, the electronic radial scan ultrasound endoscope 2 is used as the ultrasound diagnostic apparatus which is the medical instrument. However, the aspect of the ultrasound diagnostic apparatus is not limited to the present embodiment. For example, the ultrasound diagnostic apparatus may be a mechanical scan ultrasound endoscope or an electronic convex scan ultrasound endoscope including a group of ultrasound transducers provided in a sector shape. The present invention is not limited by the ultrasound scanning method.

In addition, the ultrasound diagnostic apparatus may be an ultrasound probe without an optical observation window or a capsule ultrasound sonde. In addition, the ultrasound diagnostic apparatus may be a so-called extracorporeal ultrasound diagnostic apparatus which performs ultrasound scan from the body surface toward inside of the body of the subject.

In the present embodiment, the transmission antenna 41 and the reception coil are used as the position detection means to detect the position and orientation based on the magnetic field, however, the transmission and reception relationship of the magnetic field may be reversed, and the position and orientation may be detected using acceleration or other means instead of the magnetic field.

Furthermore, in the present embodiment, though the image position/orientation detection coil 44 is disposed in the vicinity of the center of the circular ultrasound transducer array 31, the positional relationship of the two is not limited to such aspect. That is, in order to evaluate the coordinates and the orientation of the scan center of the ultrasound tomographic image from the position and the orientation of the image position/orientation detection coil 44, the relative position between the ultrasound transducer array 31 and the image position/orientation detection coil 44 has only to be fixed.

Moreover, in the present embodiment, a plurality of sheets of two-dimensional CT images and two-dimensional MRI images scanned by the X-ray three-dimensional helical CT apparatus 16 and the three-dimensional MRI apparatus 15 are used as the data configuring the group of reference image data GRD. However, three-dimensional image data previously acquired using another modality such as PET (Positoron Emission Tomography), for example, may be used as the group of reference image data GRD. Alternatively, the group of reference image data GRD may be configured of the three-dimensional image data previously acquired by a so-called extracorporeal ultrasound diagnostic apparatus, which performs ultrasound scan from outside the body.

Furthermore, in the present embodiment, the four body surface detection coils 42 wound in one axis direction are mounted to the subject 100 to acquire the position/orientation data of the four sample points A1 to A4. However, alternatively, the position/orientation data of four sample points A 1 to A4 may be acquired by having the subject 100 lie in a left lateral position, thereafter sequentially bringing one body surface detection coil into contact with the four sample points A1 to A4.

Furthermore, as for the body surface detection coils 42, the position/orientation calculation apparatus 4 calculates only the positions thereof in the present embodiment. However, the position/orientation calculation apparatus 4 may calculate the direction of the winding axis of the body surface detection coils 42 instead of the positions thereof. In addition, the position/orientation calculation apparatus 4 may calculate both the positions and direction of the winding axis of the body surface detection coils 42. With the increased degree of freedom of calculation with respect to one body surface detection coil 42 by the position/orientation calculation apparatus 4, the number of the body surface detection coils 42 can be decreased, thereby reducing the burdens of the operator and the subject 100 when the body surface detection coils 42 are mounted to the subject 100 and during the diagnosis by the ultrasound endoscope 2.

In the present embodiment, the sample points are assumed to be the xiphoid process, the left anterior superior iliac spine, the right anterior superior iliac spine, and the spinous process of vertebral body. However, the sample points are not limited to this example and may be feature points on the thoracic surface or thoracic cavity or other examples.

### (Second Embodiment)

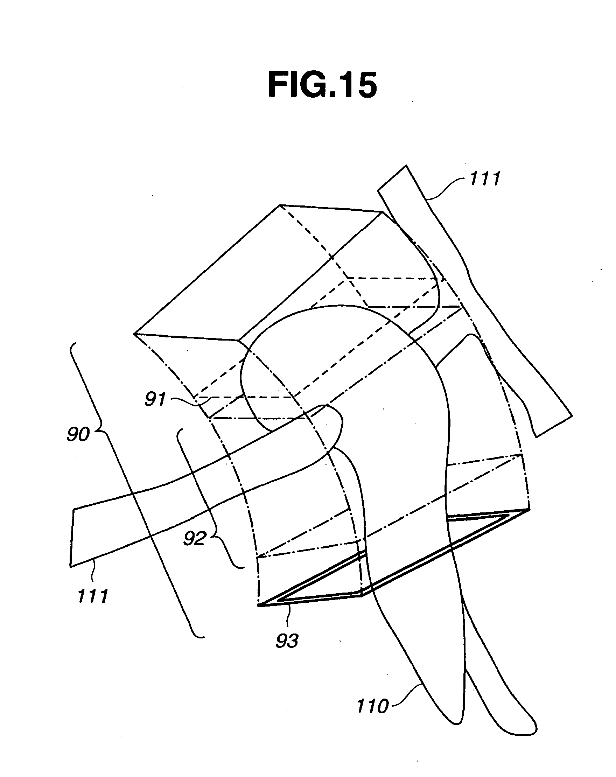

Hereinafter, the second embodiment of the present invention is described with reference to the drawings. FIG. 15 is a view describing a three-dimensional guide image of the present embodiment.

The present embodiment differs from the first embodiment only in the display aspect of the image scan history marker, but other configurations are the same. Therefore, only the different point is described below.

A medical guiding system of the present embodiment displays an image scan history marker 90 for indicating the region where the ultrasound scan has been performed is displayed in the three-dimensional guide image data, as a moving locus of the image scan range R of the ultrasound tomographic image data, as shown in FIG. 15.

The image scan history marker 90 of the present embodiment has a three-dimensional shape formed by connecting the contours of the image scan history markers 80 which are a plurality of quadrilateral planes formed by the similar method as that in the first embodiment.

The image scan history marker 90 of the present embodiment is expressed by a wireframe as a three-dimensional contour indicating the moving locus of the image scan range R of the ultrasound tomographic image data.

The wireframe of the image scan history marker 90 of the present embodiment and the region inside of the wireframe are colored by pixels of different transmissivity and color according to the change of the values of the tomographic image one-sheet saving flag 74 and the tomographic image successive saving flag 75.

That is, the image scan history marker 90 of the present embodiment distinctly expresses the following by the change in color: the moving locus of the image scan range R of the ultrasound tomographic image data; the region for which the one sheet of the ultrasound tomographic image has been saved; and the region for which the ultrasound tomographic image data have been successively saved.

For example, in the region where the ultrasound scan has been performed but for which the ultrasound tomographic image data is not saved, the wireframe of the image scan history marker 90 and the region inside of the wireframe are expressed in a semi-transparent white color. In the region for which one sheet of the ultrasound tomographic image data has been saved, the wireframe of the image scan history marker 90 and the region inside of the wireframe are expressed in green color (the reference numeral 91 in FIG. 15). In the region for which the ultrasound tomographic image data have been succesively saved, the wireframe of the image scan history marker 90 and the region inside of the wireframe is expressed in blue color (the reference numeral 92 in FIG. 15).

Moreover, the region corresponding to the position of the image scan range R of the latest ultrasound tomographic image data is expressed as a quadrilateral frame highlighted by thick black lines (the reference numeral 93 in FIG. 15).

The transmissivity and the luminance of the image scan history marker 90 can be changed to an arbitrary transmissivity and luminance by the operator's operation so that the operator can easily grasp the positional relationship between the marker and the three-dimensional model image of the organ of interest.

The above-described medical guiding system of the present embodiment not only has the same effect as that in the first embodiment, but also has a simpler expression of the image scan history marker in the three-dimensional guide image data, so that the operator can intuitively and quickly realize the history of the region where the ultrasound diagnosis has been already performed with respect to the organ of interest.

### (Third Embodiment)

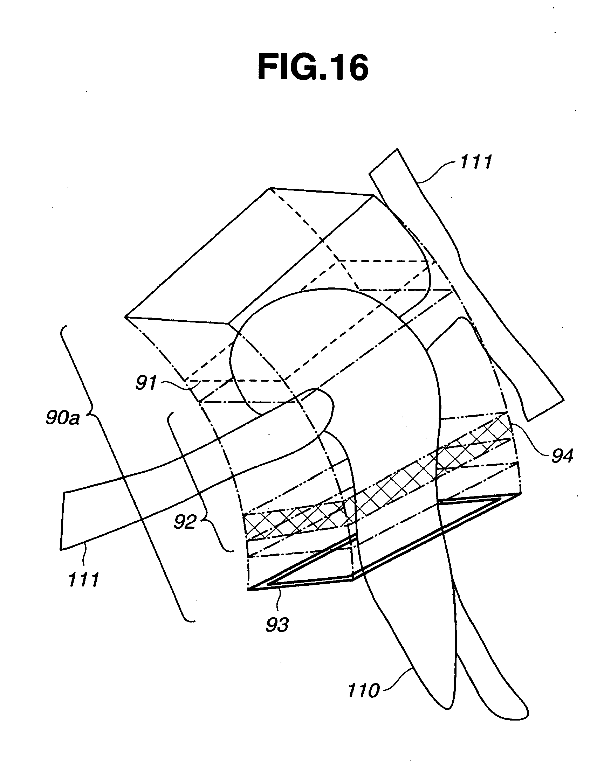

Hereinafter, the third embodiment of the present invention is described with reference to the drawings. FIG. 16 is a view describing the three-dimensional guide image of the present embodiment.

The present embodiment differs from the second embodiment only in the display aspect of the image scan history marker, but other configurations are the same. Therefore, only the different point is described below.

As in the second embodiment, a medical guiding system of the present embodiment displays an image scan history marker 90a for indicating the region where the ultrasound scan has been performed, as the moving locus of the image scan range R of the ultrasound tomographic image data in the three-dimensional guide image data.

The image scan history marker 90a of the present embodiment is expressed by a wireframe as a three-dimensional contour indicating the moving locus of the image scan range R of the ultrasound tomographic image data, as in the second embodiment. In addition, as in the second embodiment, the wireframe of the image scan history marker 90a and the region inside of the wireframe are colored by the pixels of different transmissivity and color according to the change of the values of the tomographic image one-sheet saving flag 74 and the tomographic image successive saving flag 75.

Furthermore, the image scan history marker 90a of the present embodiment is colored such that the wireframe and the region inside of the wireframe have different colors depending on the moving distance of the image scan range R during the acquisition of a predetermined number of sheets of ultrasound tomographic image data.

That is, the image scan history marker 90a of the present embodiment distinctly expresses the following by the change in color: the moving locus of the image scan range R of the ultrasound tomographic image data; the region for which one sheet of ultrasound tomographic image data has been saved; and the region for which ultrasound tomographic image data have been successively saved, and also expresses density of acquiring intervals for the acquired ultrasound tomographic image data.

For example, if the ultrasound observation apparatus 3 creates the ultrasound tomographic image data at a frame rate of 10 Hz, ten sheets of ultrasound tomographic image data are acquired per second. The three-dimensional guide image creation circuit 65 calculates the volume of the region where the ultrasound scan was performed in one second based on the moving distance of the image scan range R in one second, that is the change amount of the position and the orientation of the image position/orientation detection coil 44 and the area of the image scan range R.

When the volume of the region where the ultrasound scan was performed in one second is equal to or larger than a predetermined threshold value given in advance, the three-dimensional guide image creation circuit 65 colors the appropriate region of the image scan history marker 90a in red.

That is, in the present embodiment, when the acquiring intervals of the acquired ultrasound tomographic image data are large, that is, the moving velocity of the ultrasound transducer array 31 is too high relative to the frame rate of the ultrasound tomographic image data, the wireframe of the image scan history marker 90a and the region inside of the wireframe are expressed in red color as a warning to the operator (the reference numeral 94 in FIG. 16).

With the medical guiding system thus configured according to the present embodiment, the similar effects as those in the second embodiment are obtained and in addition, the operator can quickly recognize the region where the acquiring intervals of the ultrasound tomographic image data are coarse, thereby prevented from overlooking the region during a diagnosis.

In the diagnosis by the ultrasound endoscope, the rigid portion (ultrasound transducer array) in some cases moves largely in a mere instant during the diagnosis. For example, in a case where the operator observes the duodenal bulb while pulling the ultrasound endoscope, if the operator continues to pull the ultrasound endoscope, the rigid portion is caught in the pylorus separating between the duodenal bulb and the stomach. If the operator further continues to pull the ultrasound endoscope, the rigid portion is popped out from the pylorus, and the rigid portion sometimes moves largely by the reaction.

With the medical guiding system of the present embodiment, in a case where the ultrasound transducer array is thus moved largely in a short time, the operator can quickly and easily grasp at which position the large movement in a short time occurred and from which position the ultrasound scan is to be performed again, which can reduce the diagnosis time. Therefore, the burdens of the operator and the subject are reduced.

Note that, as for the image scan history marker 90a of the present embodiment, the region for which the ultrasound tomographic image data were saved and the density of the intervals at which the ultrasound tomographic image data were acquired are expressed at the same time by different colors. However, the two may be individually expressed and switched to be displayed by the operator's instruction.

In addition, the image scan history marker 90a may be of an aspect such that the region for which the ultrasound tomographic image data were saved is expressed only by the difference in line type, and the density of the intervals at which the ultrasound tomographic image data were acquired may be expressed by the difference in color.

In addition, the present embodiment is of an aspect in which the determination of the density of the intervals at which the ultrasound tomographic image data were acquired is made based on one threshold value. However, two or more threshold values may be set and the image scan history marker 90a may be displayed in three or more different colors.

In addition, the determination of the density of the intervals at which the ultrasound tomographic image data were acquired may be made based on the change amount of the orientation of the ultrasound transducer array per unit time, that is, an angular velocity, instead of the moving amount of the ultrasound transducer array per unit time. In addition, the determination of the density of the intervals at which the ultrasound tomographic image data were acquired may be made based on both the moving velocity and the angular velocity of the ultrasound transducer array.

Note that the present invention is not limited to the above-described embodiments, but can be adopted to a diagnosis and an operation with an endoscope such as a bronchoscope or laparoscope, or to an extracorporeal ultrasound diagnostic apparatus.

Having described the preferred embodiments of the invention referring to the accompanying drawings, it should be understood that the present invention is not limited to those precise embodiments and various changes and modifications thereof could be made by one skilled in the art without departing from the scope of the invention as defined in the appended claims.

## Claims

1. A medical guiding system (1) comprising:
a detection portion (4) for detecting at least one of a position and an orientation of an ultrasound transducer (31) which transmits and receives ultrasound to and from a subject, the ultrasound transducer (31) being provided in an ultrasound diagnostic apparatus for scanning an ultrasound tomographic image based on an ultrasound signal outputted from the ultrasound transducer (31);
a reference image storage portion (52) for storing reference image data including anatomical positional information of an organ of a human body; and
a guide image creation portion (65) for creating a guide image and outputting the created guide image to a display apparatus (8) based on the output from the detection portion (4) and the reference image data, the guide image being configured of: an anatomical model image of the organ of the human body; and an ultrasound tomographic image marker (93) showing at least one of a position and an orientation with respect to the model image of the latest ultrasound tomographic image,
**characterized in that**
the guide image being further configured of an image scan history marker (80) showing at least one of a position and an orientation with respect to the model image, of a region where the ultrasound tomographic image has been scanned in the past, and
the guide image creation portion (65) is configured to be able to create a wireframe (90, 90a) indicating a contour of a three-dimensional shape formed by connecting the regions where a plural number of the ultrasound tomographic images have been scanned in the past, in the guide image.

2. The medical guiding system (1) according to claim 1, wherein the ultrasound diagnostic apparatus successively scans the ultrasound tomographic images, and the guide image creation portion (65) calculates a moving velocity of the ultrasound transducer (31) based on the output from the detection portion (4), and when the moving velocity is equal to or greater than a predetermined value, a region inside the wireframe (90, 90a) is displayed in a different colour with respect to a display of other regions where the moving velocity is within the predetermined value.

3. The medical guiding system (1) according to claim 1 or 2, further comprising
an image scan region storage portion (53) for storing image scan region data (70) configured of information on at least one of positions and orientations with respect to the model image, of regions where all the ultrasound tomographic images have been scanned, wherein the guide image creation portion (65) creates an image scan history image to output the created image scan history image to the display apparatus (8) based on the image scan region data (70), the image scan history image indicating at least one of the positions and the orientations with respect to the model image of the regions where all the ultrasound tomographic images have been scanned.

4. The medical guiding system (1) according to claim 3, further comprising:
a tomographic-image-for-saving storage portion (55) for storing at least one of the ultrasound tomographic images in association with information on at least one of the position and the orientation of the region where the ultrasound tomographic image has been scanned; and
an ultrasound tomographic image reproducing portion for selecting and reading a predetermined one of the ultrasound tomographic images from the tomographic-image-for-saving storage portion (55) to reproduce and output the ultrasound tomographic image to the display apparatus (8) after the diagnosis;
wherein the guide image creation portion (65) creates an index indicating at least one of the position and the orientation with respect to the model image, of the region where the predetermined one ultrasound tomographic image reproduced by the ultrasound tomographic image reproducing portion has been scanned, to superimpose the index on the image scan history image.

## Patentansprüche

1. Medizinisches Führungssystem (1) aufweisend:
einen Erfassungsteil (4) zum Erfassen einer Position und/oder einer Orientierung eines Ultraschallwandlers (31), der Ultraschall zu einem Subjekt sendet und von diesem empfängt, wobei der Ultraschallwandler (31) in einer Ultraschalldiagnosevorrichtung zum Abtasten eines Ultraschalltomographiebildes basierend auf einem von dem Ultraschallwandler (31) ausgegebenem Ultraschallsignal bereitgestellt ist;
einen Referenzbildspeicherteil (52) zum Speichern von Referenzbilddaten enthaltend anatomische Positionsinformation eines Organ eines lebenden Körpers; und
einen Führungsbilderzeugungsteil (65) zum Erzeugen eines Führungsbildes und Ausgeben des erzeugten Führungsbildes an eine Anzeigevorrichtung (8) basierend auf der Ausgabe des Erfassungsteils (4) und der Referenzbilddaten, wobei das Führungsbild konfiguriert ist aus: einem anatomischen Modelbild des Organs des menschlichen Körpers; und einer Ultraschalltomographiebildmarkierung (93), die eine Position und/oder eine Orientierung hinsichtlich des Modelbildes des neuesten Ultraschalltomographiebildes zeigt;
**dadurch gekennzeichnet, dass**
das Führungsbild ferner konfiguriert ist aus einer
Bildabtastungsentwicklungsmarkierung (80), die eine Position und/oder Orientierung hinsichtlich des Modelbildes, eines Bereichs, in dem das Ultraschalltomographiebild in der Vergangenheit abgetastet wurde, zeigt, und
das Führungsbilderzeugungsteil (65) dazu eingerichtet ist, in der Lage zu sein, ein Drahtmodel (90, 90a), das eine Kontur einer dreidimensionalen Form anzeigt, die durch Verbinden der Bereiche in denen eine Mehrzahl der Ultraschalltomographiebilder in der Vergangenheit abgetastet wurden, in dem Führungsbild zu erzeugen.

2. Medizinisches Führungssystem (1) nach Anspruch 1, bei dem die Ultraschalldiagnosevorrichtung nacheinander die Ultraschalltomographiebilder abtastet, und der Führungsbilderzeugungsteil (65) eine Bewegungsgeschwindigkeit des Ultraschallwandlers (31) basierend auf der Ausgabe des Erfassungsteils (4) berechnet, wobei, falls die Bewegungsgeschwindigkeit gleich oder größer als ein vorbestimmter Wert ist, wird ein Bereich innerhalb des Drahtmodels (90a, 90a) mit einer anderen Farbe hinsichtlich einer Anzeige anderer Bereiche, in denen die Bewegungsgeschwindigkeit innerhalb des vorbestimmten Werts ist, anzeigt.

3. Medizinisches Führungssystem (1) nach Anspruch 1 oder 2, ferner aufweisend
einen Bildabtastungsbereichspeicherteil (53) zum Speichern von Bildabtastungsbereichsdaten (70), die aus Information über Positionen und/oder Orientierungen hinsichtlich des Modelbildes, von Bereichen, in denen alle Ultraschalltomographiebilder abgetastet wurden, bestehen, wobei der Führungsbilderzeugungsteil (65) ein Bildabtastungsentwicklungsbild zum Ausgeben des erzeugten Bildabtastungsentwicklungsbildes an die Anzeigevorrichtung (8) basierend auf den Bildabtastungsbereichsdaten (70) erzeugt, wobei das Bildabtastungsentwicklungsbild Positionen und/oder Orientierungen hinsichtlich des Modelbildes der Bereiche, in denen alle Ultraschalltomographiebilder abgetastet wurden, anzeigt.

4. Medizinisches Führungssystem (1) nach Anspruch 3, ferner aufweisend:
einen Tomographiebild-zum-Sichern-Speicherteil (55) zum Speichern von mindesten einem der Ultraschalltomographiebilder in Verbindung mit Information über die Position und/oder die Orientierung des Bereichs, in dem das Ultraschalltomographiebild abgetastet wurde;
einen Ultraschalltomographiebildnachbildungsteil zum Auswählen und Auslesen eines vorbestimmten der Ultraschalltomographiebilder von dem Tomographiebild-zum-Sichern-Speicherteil (55) zum Nachbilden und Ausgeben des Ultraschalltomographiebildes an die Anzeigevorrichtung (8) nach der Diagnose;
wobei der Führungsbilderzeugungsteil (65) einen Index erzeugt, der die Position und/oder die Orientierung hinsichtlich des Modelbildes, der Region, in der das vorbestimmte eine Ultraschalltomographiebild, welches durch den Ultraschalltomographiebildnachbildungsteil abgetastet wurde, anzeigt, um den Index dem Bildabtastungsentwicklungsbild zu überlagern.

## Revendications

1. Système (1) de guidage médical comprenant :
une partie (4) de détection destinée à détecter au moins l'une parmi une position et une orientation d'un transducteur à ultrasons (31) qui émet et reçoit des ultrasons vers et depuis un sujet, le transducteur à ultrasons (31) étant prévu dans un appareil de diagnostic par ultrasons destiné à balayer une image tomographique ultrasonore sur la base d'un signal ultrasonore délivré en sortie depuis le transducteur à ultrasons (31) ;
une partie (52) de stockage d'images de référence destinée à stocker des données d'images de référence comprenant des informations de position anatomique d'un organe d'un corps humain ; et
une partie (65) de création d'image de guidage destinée à créer une image de guidage et à délivrer en sortie l'image de guidage créée vers un appareil d'affichage (8) sur la base de la sortie de la partie (4) de détection et des données d'images de référence, l'image de guidage étant configurée à partir : d'une image modèle anatomique de l'organe du corps humain ; et d'un marqueur (93) d'image tomographique ultrasonore montrant au moins l'une parmi une position et une orientation par rapport à l'image modèle de la dernière image tomographique ultrasonore,
**caractérisé en ce que**
l'image de guidage est en outre configurée à partir d'un marqueur (80) d'historique de balayage d'image montrant au moins l'une parmi une position et une orientation par rapport à l'image modèle, d'une région dans laquelle l'image tomographique ultrasonore a été balayée par le passé, et
la partie (65) de création d'image de guidage est configurée pour être capable de créer une représentation en fil de fer (90, 90a) indiquant un contour d'une forme en trois dimensions formée en connectant les régions dans lesquelles un nombre multiple d'images tomographiques ultrasonores ont été balayées par le passé, dans l'image de guidage.

2. Système (1) de guidage médical selon la revendication 1, dans lequel l'appareil de diagnostic par ultrasons balaye successivement les images tomographiques ultrasonores, et la partie (65) de création d'image de guidage calcule une vitesse de déplacement du transducteur à ultrasons (31) sur la base de la sortie de la partie (4) de détection, et lorsque la vitesse de déplacement est égale ou supérieure à une valeur prédéterminée, une région à l'intérieur de la représentation en fil de fer (90, 90a) est affichée dans une couleur différente par rapport à un affichage des autres régions dans lesquelles la vitesse de déplacement se situe à l'intérieur de la valeur prédéterminée.

3. Système (1) de guidage médical selon la revendication 1 ou 2, comprenant en outre
une partie (53) de stockage de région de balayage d'image destinée à stocker des données de région de balayage d'image (70) configurée à partir d'informations sur au moins l'une parmi les positions et les orientations par rapport à l'image modèle, des régions dans lesquelles toutes les images tomographiques ultrasonores ont été balayées, dans lequel la partie (65) de création d'image de guidage crée une image d'historique de balayage d'image pour délivrer en sortie l'image d'historique de balayage d'image créée vers l'appareil d'affichage (8) sur la base des données (70) de région de balayage d'image, l'image d'historique de balayage d'image indiquant au moins l'une parmi les positions et les orientations par rapport à l'image modèle des régions dans lesquelles toutes les images tomographiques ultrasonores ont été balayées.

4. Système (1) de guidage médical selon la revendication 3, comprenant en outre :
une partie (55) de stockage d'images tomographiques pour sauvegarde destinée à stocker au moins l'une des images tomographiques ultrasonores en association avec des informations sur au moins l'une parmi la position et l'orientation de la région dans laquelle l'image tomographique ultrasonore a été balayée ; et
une partie de reproduction d'image tomographique ultrasonore destinée à sélectionner et à lire l'une prédéterminée parmi les images tomographiques ultrasonores à partir de la partie (55) de stockage d'images tomographiques pour sauvegarde pour reproduire et délivrer en sortie l'image tomographique ultrasonore vers l'appareil d'affichage (8) après le diagnostic ;
dans lequel la partie (65) de création d'image de guidage crée un index indiquant au moins l'une parmi la position et l'orientation par rapport à l'image modèle, de la région dans laquelle l'image tomographique ultrasonore prédéterminée reproduite par la partie de reproduction d'image tomographique ultrasonore a été balayée, pour superposer l'index sur l'image d'historique de balayage d'image.
